(19) Europäisches Patentamt
European Patent Office
Office européen des brevets

(11) EP 1 161 685 B1

(12) **FASCICULE DE BREVET EUROPEEN**

(45) Date de publication et mention de la délivrance du brevet:
**09.01.2008 Bulletin 2008/02**

(21) Numéro de dépôt: **00910933.1**

(22) Date de dépôt: **14.03.2000**

(51) Int Cl.:
***G01N 33/58*** *(2006.01)* ***G01N 33/533*** *(2006.01)*

(86) Numéro de dépôt international:
**PCT/FR2000/000608**

(87) Numéro de publication internationale:
**WO 2000/055630 (21.09.2000 Gazette 2000/38)**

(54) **REDUCTION DE L'EXTINCTION DE FLUORESCENCE LORS D'UN DOSAGE**

VERKLEINERUNG DER FLUORESZENZLÖSCHUNG WÄHREND EINER BESTIMMUNG

METHOD FOR REDUCING FLUORESCENCE QUENCHING IN BIOASSAYS

(84) Etats contractants désignés:
**AT BE CH CY DE DK ES FI FR GB GR IE IT LI LU MC NL PT SE**

(30) Priorité: **15.03.1999 FR 9903150**

(43) Date de publication de la demande:
**12.12.2001 Bulletin 2001/50**

(73) Titulaire: **CIS BIO INTERNATIONAL**
**91400 Saclay (FR)**

(72) Inventeurs:
- **MATHIS, Gérard**
  **F-30200 Bagnols sur Cèze (FR)**
- **BAZIN, Hervé**
  **F-30400 Villeneuve les Avignon (FR)**
- **TRINQUET, Eric**
  **F-30130 Pont Saint Esprit (FR)**

(74) Mandataire: **Gillard, Marie-Louise**
**Cabinet Beau de Loménie,**
**158, rue de l'Université**
**75007 Paris (FR)**

(56) Documents cités:
**EP-A- 0 321 353** **EP-A- 0 340 675**
**EP-A- 0 851 228** **FR-A- 2 769 315**
**US-A- 4 748 111**

- **E LOPEZ, C CHYPRE, B ALPHA, G MATHIS: "Europium(III) Trisbipyridine Cryptate Label for the Time-Resolved Fluorescence Detection of Polymerase Chain Reaction Products Fixed on a Solid Support" CLINICAL CHEMISTRY, vol. 39, no. 2, 1993, XP002125037 cité dans la demande**
- **I HEMMILÄ: "Fluoroimmunoassays and Immunofluorometric Assays" CLINICAL CHEMISTRY, vol. 31, no. 3, 1985, pages 359-370, XP002125038 cité dans la demande**
- **G MATHIS: "Rare Earth Cryptates and Homogeneous Fluoroimmunoassays with Human Sera" CLINICAL CHEMISTRY, vol. 39, no. 9, 1993, pages 1953-1959, XP002125039 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne l'utilisation d'un conjugué fluorescent comprenant un oligonucléotide lié à un cryptate de terre rare pour réduire l'extinction de fluorescence due au milieu de mesure, dans un dosage par fluorescence d'un analyte mettant en oeuvre au moins un marqueur fluorescent.

L'avancée des connaissances en biologie crée un besoin croissant pour des méthodes de diagnostic permettant de suivre ou de quantifier des biomolécules.

Dans le même temps, on observe une désaffection vis à vis des marqueurs radioactifs qui sont généralement impliqués dans les méthodes de dosage de référence. D'une façon générale, on cherche actuellement à remplacer les traceurs radioactifs par d'autres marqueurs et principalement par des marqueurs fluorescents. L'utilisation de marqueurs fluorescents dans des conditions idéales permet d'obtenir des sensibilités élevées théoriquement équivalentes à celles obtenu par les traceurs radioactifs.

Dans la pratique, les performances des traceurs fluorescents sont limitées, d'une part, par la présence d'un bruit de fond souvent élevé et, d'autre part, par le fait qu'ils sont généralement très sensibles au changements dans leur environnement. Des petites modifications du pH, de la polarité, de la présence d'oxygène dissous, de la proximité d'atomes lourds (iode par exemple) ou de groupes absorbants peuvent modifier leur rendement quantique (dans le sens d'une exaltation ou d'une extinction) ou déplacer la longueur d'onde de l'émission.

Il est connu que l'interaction avec des protéines présentes dans le sérum provoque souvent une extinction (« quenching ») de la fluorescence.

Les problèmes inhérents aux méthodes d'analyse par mesure de la fluorescence sont répertoriés dans un article de revue (1. Hemmilä, Clin. Chem. 31/3, 359-370 (1985)).

Les problèmes inhérents au bruit de fond provenant de la fluorescence intrinsèque des protéines ainsi que des autre biomolécules présentes dans les échantillons biologiques peut être résolu par l'utilisation de marqueurs fluorescents formés par des complexes de terres rares (principalement l'Europium) qui permettent une sélection temporelle du signal spécifique. Les durées de vie particulièrement longues (0,1ms à 1 ms environ) qui caractérisent les complexes d'Europium permettent, à l'aide d'une mesure en temps résolu, de s'affranchir du bruit de fond provenant, par exemple, des protéines sériques qui lui, est caractérisé par une durée de vie relativement courte (environ 4ns).

Le marquage indirect d'acides nucléiques par un cryptate trisbipyridine-Europium [TBP-($Eu^{3+}$)] (cryptate décrit dans le brevet EP 0321353) a été réalisé par l'intermédiaire d'un anticorps anti-DNP marqué par ce cryptate, le groupe dinitrophényle (DNP) étant introduit à l'extrémité 5' d'oligonucléotides synthétiques (E. Lopez et al., Clin. Chem. 39/2, 196-201 (1993)).

L'utilisation d'anticorps marqués à l'aide d'un cryptate TBP-($Eu^{3+}$) s'est par ailleurs étendu au domaine de l'immunodiagnostic. L'utilisation d'un cryptate comme marqueur a permis de développer des immuno-essais de type homogène basés sur une mesure de fluorescence en temps résolu associé à un transfert d'énergie non radiatif (G.Mathis et al., Clin. Chem, 39, 1251 (1993)).

Un format de type homogène présente l'intérêt considérable de pouvoir suivre en temps réel la cinétique de formation d'un complexe immunologique, mais ne permet cependant pas de s'affranchir des interactions éventuellement défavorables entre le marqueur et les molécules présentent dans un milieu biologique (extinction de la fluorescence).

Dans un milieu sérique, on peut obtenir une restauration des propriétés photophysiques, et notamment de la durée de vie, en ajoutant des ions fluorures au milieu comme décrit dans la demande WO92/01124.

On a maintenant trouvé que le fait de conjuguer une molécule de cryptate de terre rare à une chaîne oligonucléotidique permet d'obtenir un conjugué fluorescent cryptate-oligonucléotide qui présente des propriétés photophysiques nouvelles et inattendues.

Ledit conjugué présente la propriété avantageuse d'être moins sensible, comparativement au cryptate seul, au phénomène d'extinction de la fluorescence résultant d'une interaction avec des molécules présentes dans le milieu.

Cette observation présente un grand intérêt puisqu'elle permet de réaliser des mesures de fluorescence dans des milieux biologiques sans utilisation d'un adjuvant comme les ions fluorures.

Les conjugués cryptate-oligonucléotides constituent donc de nouveaux marqueurs, qui peuvent être couplés à une molécule biologique ayant un rôle de reconnaissance et pouvant se lier à un partenaire.

Le conjugué cryptate-oligonucléotide couplé à un récepteur tel qu'un anticorps ou la streptavidine garde ses propriétés photophysiques (résistance à l'extinction) et présente des propriétés avantageuses en comparaison des conjugués cryptate-anticorps ou cryptate-streptavidine.

L'invention concerne donc, selon un premier aspect, un procédé de réduction de l'extinction de fluorescence due au milieu de mesure, dans un dosage par fluorescence d'un analyte mettant en oeuvre au moins un marqueur fluorescent, caractérisé en ce qu'on introduit dans le milieu de mesure un conjugué fluorescent comprenant un oligonucléotide lié à un cryptate de terre rare.

Dans un aspect avantageux, le conjugué fluorescent est lui-même utilisé comme seul marqueur ou comme l'un des marqueurs fluorescents dans le dosage.

Par « analyte », on entend dans la présente description toute substance ou groupe de substances, ainsi que ses ou leurs analogues, que l'on souhaite détecter et/ou déterminer.

Le procédé selon l'invention trouve une application importante dans les procédés de dosage dits par compétition ou par excès, en phase homogène.

Dans la suite de la description, la notion de « cryptate » ainsi que la nomenclature des macrocycles et polycycles utilisables sont telles que définies par J.M. Lehn dans Struct. Bonding (Berlin), 16, 1, 1973 et dans Acc. Chem. Res. 11, 49, (1978).

Par « oligonucléotide », on entend dans la présente description :

- soit un enchaînement d'unités ribonucléotides ou désoxyribonucléotides liées entre elles par des liaisons de type phosphodiester ;
- soit un enchaînement d'unités ribonucléotides ou désoxyribonucléotides ou d'unités analogues de nucléotides modifiées sur le sucre ou sur la base et liées entre elles par des liaisons internucléotidiques naturelles de type phosphodiester, une partie des liaisons internucléotidiques étant éventuellement remplacée par des liaisons phosphonate, phosphoramide ou phosphorothioate. Ces différentes familles d'oligonucléotides sont décrites dans Goodchild, Bioconjugate Chemistry, 1(3), May/June 1990, 77-99 ;
- soit un enchaînement comprenant à la fois des unités ribonucléotides ou désoxyribonucléotides liées entre elles par des liaisons de type phosphodiester et des unités analogues de nucléosides liées entre elles par des liaisons amides, communément dénommés « PNA » (en anglais « peptide nucleic acid »), tel que décrit dans M. Egholm et al., J. Am. Chem. Soc., 1992, 114, 1895-1897 ; de tels composés sont par exemple décrits dans R. Vinayak et al., Nucleoside & Nucleotide, 1997, 16 (7-9), 1653-1656.

L'utilisation de chacun de ces types d'oligonucléotides constitue un aspect avantageux de l'invention.

On entend par « analogue » de nucléotide ou de nucléoside un nucléotide/nucléoside comportant au moins une modification portant sur le sucre ou la nucléobase ou une combinaison de ces modifications. A titre d'exemple, on peut citer les modifications suivantes :

I. Modifications concernant le sucre (analogues de nucléotides ou de nucléosides) :

1°) La partie sucre peut être modifiée en ce que la configuration des hydroxyles (libres ou engagés dans un pont phosphate) est différente de la configuration naturelle (qui est respectivement β-D-*érythro* en série ADN et β-D-*ribo* en série ARN) comme dans les analogues ayant le squelette β-D-*arabino*-pentofuranoside ou β-D-*xylo*-pentofuranoside, par exemple.

2°) La structure peut être modifiée en ce que les liaisons internucléotidiques sont de type 2' → 5', tel que dans le cas des dérivés β-D-*ribo*-pentofuranoside-2'-phosphate ou 3'-désoxy-β-D-*érythro*-pentofuranoside-2'-phosphate.

Il existe des nucléotides dont la structure regroupe les deux modifications précédentes, tel que le β-D-*xylo*-pentofuranoside-2'-phosphate.

3°) La structure peut différer du modèle naturel en ce que la configuration du carbone 4' est opposée, c'est le cas α-L-*thréo*-pentofuranoside-3'-phosphate. La différence peut porter sur la configuration du carbone en 1' (position anomérique) c'est le cas du α-D-*érythro*-pentofuranoside-3'-phosphate. Il existe des nucléotides/nucléosides dont la structure regroupe les deux modifications précédentes, tel que le β -L-*thréo*-pentofuranoside-3'-phosphate.

4°) La structure peut différer du modèle naturel en ce que l'oxygène en 4' est remplacé par un carbone (analogue carbocyclique) ou par un soufre tel que le 4'-Thio-β-D-*érythro*-pentofuranoside-3'-phosphate.

5°) La structure peut différer du modèle naturel en ce que l'un des hydroxyle du sucre est alkylé, par exemple dans le squelette 2'-O-Alkyl-β-D-*ribo*-pentofuranoside-3'-phosphate, le groupe alkyle pouvant être par exemple le groupe méthyle ou allyle.

6°) La structure peut différer du modèle naturel en ce que seule la partie sucre est conservée comme dans le 1,2-didésoxy-D-*érythro*-pentofuranose-3-phosphate, ou en ce que le sucre est remplacé par un polyol comme le propanediol.

II. Modifications concernant la nucléobase (analogues de nucléotide) :

1°) La nucléobase peut être modifiée en ce que les substituants des bases naturelles sont modifiés comme dans la 2,6-diaminopurine, l'hypoxanthine, la 4-Thio-thymine, le 4-Thio-uracil, ou le 5-Ethynyl-uracil.

2°) Les positions des substituants peuvent être permutées par rapport aux bases naturelles tel que dans l' Isoguanosine ou l'Isocytosine.

3°) Un atome d'azote de la nucléobase peut être remplacé par un carbone comme dans la 7-Déaza-guanosine, la 7-Déaza-adénine.

Par ailleurs, comme mentionné ci-dessus, les liaisons entre les unités sucres ou leurs analogues peuvent également être modifiées, par exemple en remplaçant un ou plusieurs des atomes d'oxygène de la liaison phosphodiester naturelle par un carbone (série phosphonates), un azote (série phosphoramides), ou un soufre (phosphorothioates).

Avantageusement, l'oligonucléotide du conjugué selon l'invention est constitué d'unités ribonucléotides ou désoxyribonucléotides, dont l'une peut comporter un groupe fonctionnel introduit ou généré sur ladite unité ou un groupe fonctionnel introduit à l'aide d'un bras d'espacement lié au groupement phosphate terminal en position 3' ou 5'.

Selon un aspect préféré, ladite unité est l'unité 5' terminale ou 3' terminale.

L'oligonucléotide utilisable selon l'invention comprendra de préférence un enchaînement de 5 à 50 nucléotides ou un enchaînement de 5 à 50 nucléotides et analogues de nucléotides ou de nucléosides tels que définis ci-dessus.

Selon un aspect particulier de l'invention, on utilisera un oligonucléotide constitué d'un enchaînement d'unités ribonucléotides ou désoxyribonucléotides liées entre elles par des liaisons de type phosphodiester, et d'unités analogues de nucléosides liées entre elles par des liaisons amide, ledit oligonucléotide comprenant au moins 5 liaisons internucléotidiques de type phosphodiester à l'extrémité destinée à être liée au cryptate.

Selon un aspect préféré, ledit cryptate de terre rare est constitué d'au moins un sel de terre rare complexé par un composé macropolycyclique de formule

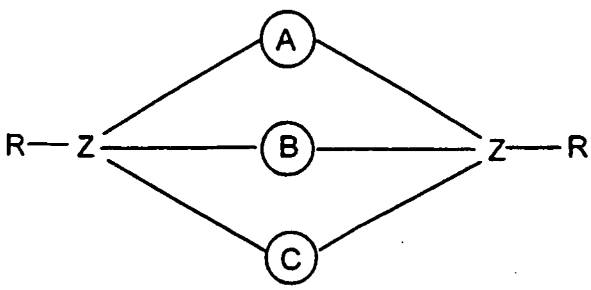

I

dans laquelle Z est un atome ayant 3 ou 4 valences, R est rien ou représente l'hydrogène, le groupe hydroxy, un groupe amino ou un radical hydrocarboné, les radicaux bivalents Ⓐ, Ⓑ et Ⓒ, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ et Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

En particulier, ledit cryptate de terre rare répond à la formule (I) dans laquelle le motif moléculaire est choisi parmi la phénanthroline, l'anthracène, le benzène, le naphtalène, les bi- et ter-phényle, l'azobenzène, l'azopyridine, la pyridine, les bipyridines, les bisquinoléines et les composés de formules ci-après :

$$- C_2H_4 - X_1 - C_6H_4 - X_2 - C_2H_4 -$$

$$- C_2H_4 - X_1 - CH_2 - C_6H_4 - CH_2 - X_2 - C_2H_4 -$$

$X_1$ et $X_2$ pouvant être identiques ou différents désignent l'oxygène, l'azote ou le soufre,

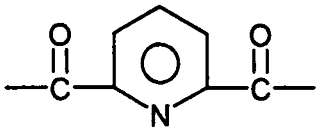

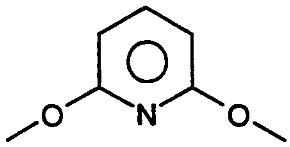

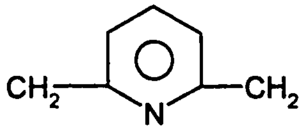

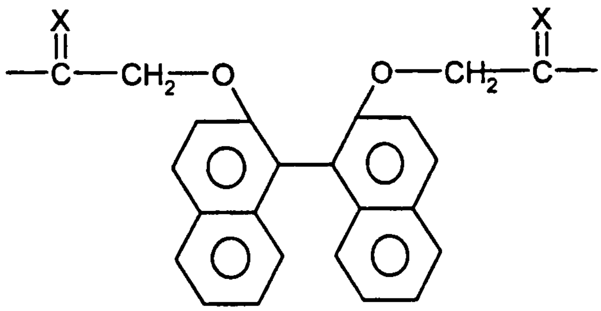

X étant l'oxygène ou l'hydrogène.

Avantageusement, ledit cryptate de terre rare est constitué d'un sel de terre rare complexé par l'un des composés macrocycliques ci-après :

(22)phénanthroline ; (22)phénanthroline amide ; (22)anthracène ; (22)anthracène amide ; (22)bi-isoquinoléine ; (22) biphényl-bis-pyridine ; (22)bipyridine ; (22)bi-pyridine amide ; les macropolycycles tris-bipyridine, tris-phénanthroline, phénanthroline-bis-bipyridine, bi-isoquinoléine-bis-bipyridine, bis-bipyridine diphénylbipyridine.

De tels composés sont par exemple décrits dans le brevet EP 180 492.

On peut également utiliser des composés macropolycycliques cryptates complexant des ions de terre rare dans lesquels le motif moléculaire est choisi parmi les bipyrazines, les bipyrimidines et les hétérocycles azotés comportant des groupes N-oxydes.

Des composés macropolycycliques à unités bipyrazines sont décrits dans F. Bodar-Houillon et al., New J. Chem., 1996, 20, 1041-1045.

Des composés macropolycycliques à unités bipyrimidines sont décrits dans J. M. Lehn et al., Helv. Chim. Acta, 1992, 75, 1221.

Des composés macropolycycliques comprenant des hétérocycles azotés comportant des groupes N-oxydes sont décrits dans J.M. Lehn et al., Helv. Chim. Acta, 1991, 74, 572.

Selon un autre aspect avantageux, ledit cryptate de terre rare est constitué d'au moins un sel de terre rare complexé par un composé macropolycyclique répondant à l'une des formules II ou III ci-après :

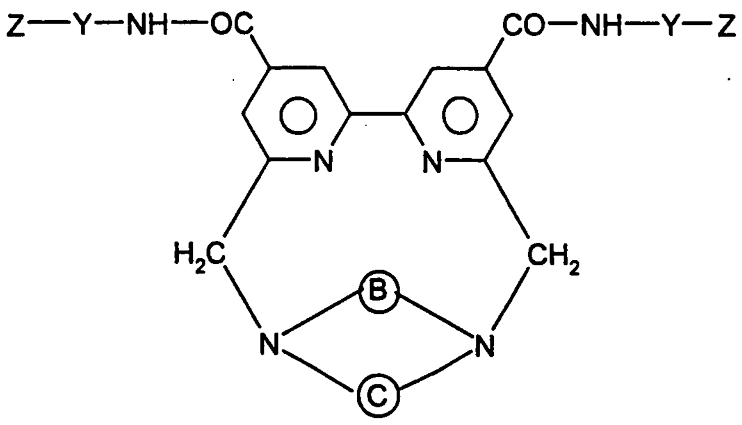

II

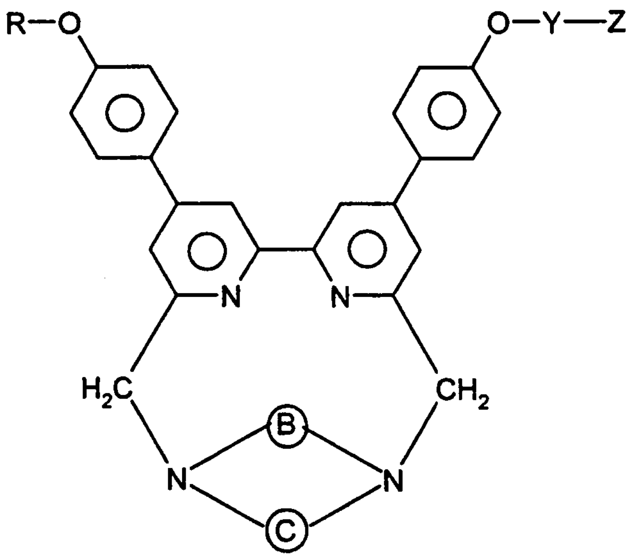

III

dans lesquels :

- le cycle de formule

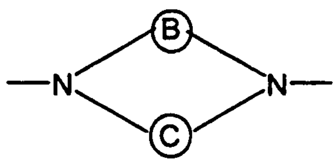

est l'un des cycles suivants :

1)

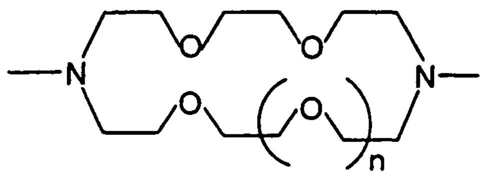

n = 0 ou 1

macrocycle [$N_2O_4$] ou cycle (22)

macrocycle [$N_2O_3$] ou cycle (21)

2)

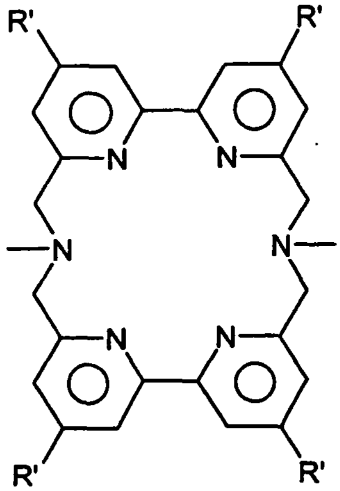

macrocycle bis-bipyridine

- Y est un groupe ou un bras d'espacement qui est constitué par un radical organique bivalent, choisi parmi les groupes alkylène linéaires ou ramifiés en $C_1$ à $C_{20}$ contenant éventuellement une ou plusieurs doubles liaisons et/ou éventuellement contenant un ou plusieurs hétéroatomes tels que l'oxygène, l'azote, le soufre, le phosphore ou un ou plusieurs groupe(s) carbamoyle ou carboxamido ; parmi les groupes cycloalkylène en $C_5$ à $C_8$ ou parmi les groupes arylène en $C_6$ à $C_{14}$, lesdits groupes alkylène, cycloalkylène ou arylène étant éventuellement substitués par des groupes alkyle, aryle ou sulfonate ;
- Z est un groupe fonctionnel susceptible de se lier de façon covalente avec une substance biologique ;
- R est un groupe méthyle ou représente le groupe -Y-Z ;
- R' est l'hydrogène ou un groupe -COOR" dans lequel R" est un groupe alkyle en $C_1$ à $C_{10}$ et représente de préférence le groupe méthyle, éthyle ou tertiobutyle ou bien R' est un groupe -CO-NH-Y-Z.

Selon un aspect préféré, le cryptate de terre rare du conjugué fluorescent utilisé selon l'invention est un cryptate d'europium.

Dans un aspect avantageux, ledit cryptate de terre rare est le cryptate d'europium Eu trisbipyridine ou Eu [bis-diéthoxybipyridine.bipyridine].

Le cryptate de terre rare est de préférence lié de manière covalente à l'oligonucléotide soit directement, soit par l'intermédiaire d'un bras d'espacement.

Par « liaison directe », on entend la liaison du marqueur fluorescent sur un groupe fonctionnel préalablement introduit ou généré sur un ou plusieurs atomes d'une base ou d'une unité pentofuranose de l'oligonucléotide.

Dans la présente description, on désigne par groupe fonctionnel toute fonction portée par la partie nucléotidique ou introduite sur cette partie par toute méthode connue par l'homme du métier et capable de se lier par liaison covalente, directement ou après activation avec une fonction présente sur le cryptate ou sur le bras espaceur porté par le cryptate. De tels groupes fonctionnels sont notamment les fonctions $NH_2$, COOH, CHO, OH ou SH ainsi que les fonctions capables de donner des liaisons covalentes par substitution (halogénures, sulfonates, époxyde) ou par addition (double liaisons type maléïmide). Ces fonctions sont généralement portées par une chaîne hydrocarbonée elle-même reliée à la partie nucléotidique.

Des méthodes d'introduction de ces groupes fonctionnels sont notamment décrites dans C. Kessler, Nonisotopic probing, Blotting and Sequencing, 2nd edition, L.J. Kricka (1995), Ed. Academic press Ltd., Londres, p. 66-72.Selon un

aspect préféré de l'invention, le cryptate de terre rare est lié à l'oligonucléotide par l'intermédiaire d'un bras d'espacement. On entend par « bras d'espacement » tout moyen permettant de lier de façon covalente l'oligonucléotide avec le cryptate au niveau d'un phosphate terminal, d'un atome d'une base purique ou pyrimidique ou d'un atome du sucre.

Dans un aspect avantageux, ledit bras d'espacement est constitué par un radical organique bivalent, choisi parmi les groupes alkylène linéaires ou ramifiés en $C_1$-$C_{20}$, contenant éventuellement une ou plusieurs doubles liaisons ou triples liaisons et/ou contenant éventuellement un ou plusieurs hétéroatomes, tels que l'oxygène, l'azote, le soufre, le phosphore ou un ou plusieurs groupe(s) carbamoyle ou carboxamido ; les groupes cycloalkylène en $C_5$-$C_8$ et les groupes arylène en $C_6$-$C_{14}$, lesdits groupes alkylène, cycloalkylène ou arylène étant éventuellement substitués par des groupes alkyle, aryle ou sulfonate.

En particulier, le bras d'espacement est choisi parmi les groupes de formules :

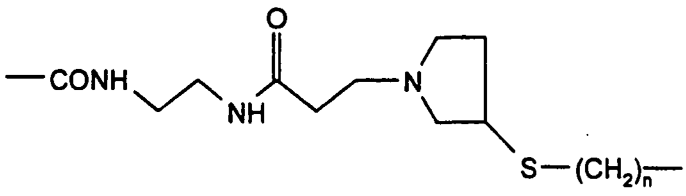

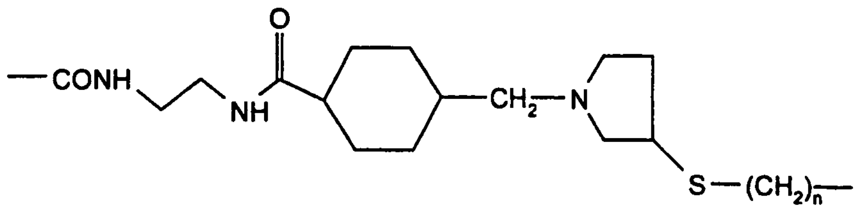

dans lesquelles n = 2 à 6, et $-CONH-(CH_2)_6-$,
la liaison via le groupe -CONH ayant lieu au niveau du cryptate.

Selon un aspect ultérieur de l'invention, le conjugué fluorescent est lié de manière covalente à l'un des membres d'un couple de molécules capable de se lier spécifiquement entre elles, comme par exemple un couple antigène/anticorps, ligand/récepteur cellulaire, biotine/avidine, acide nucléique (notamment un ARN ou un ADN mono- ou bi-caténaire, ou un oligonucléotide mono- ou bi-caténaire) et l'acide nucléique comportant des bases complémentaires à celui-ci.

Selon un aspect du procédé selon l'invention, la fluorescence du conjugué fluorescent utilisé comme marqueur est émise directement par le marqueur fluorescent, après excitation à une longueur d'onde donnée.

Selon un autre aspect du procédé selon l'invention, on met en oeuvre dans le dosage, outre ledit conjugué fluorescent, un autre marqueur fluorescent. Dans ce cas, la fluorescence mesurée dans le dosage est émise de manière indirecte par un transfert d'énergie non radiatif entre le conjugué après excitation dit « composé donneur » et une autre molécule fluorescente dite « composé accepteur ».

Dans ce cas particulier, les conditions suivantes sont remplies :

- d'une part, le composé fluorescent accepteur possède un spectre d'absorption qui recouvre au moins partiellement le spectre d'émission du donneur et présente une absorbance molaire élevée dans cette zone de recouvrement, et un spectre d'émission dans une gamme de longueur d'ondes où le donneur présente une émission intrinsèque faible ;
- d'autre part, l'accepteur et le donneur se situent à proximité l'un de l'autre, l'orientation de leurs dipoles de transition étant approximativement parallèles.

Le principe de la technique de transfert d'énergie non radiatif est décrit notamment dans G. Mathis et al., Clin. Chem., 1993, 39, 1953-1959.

Le cryptate de terre rare lié à l'oligonucléotide au sein du conjugué, qui est le composé fluorescent donneur, peut être dans ce cas un cryptate d'europium, et le composé fluorescent accepteur peut être par exemple choisi parmi l'allophycocyanine, l'allophycocyanine B, la C phycocyanine ou la R phycocyanine.

L'invention est illustrée par les exemples ci-après, dans lesquels on utilisera les abréviations suivantes :

BSA : sérum albumine bovine

DTT : dithiothréitol
SMCC : ester N-hydroxysuccinimide de l'acide 4-(N-maléimidométhyl) cyclohexane-1-carboxylique
SMP : ester N-hydroxysuccinimide de l'acide 3-maléimidopropionique
SPDP : N-succinimidyl-3-(2-pyridyldithio)propionate
SVNN : sérum de veau nouveau-né
TEAB : hydrogénocarbonate de triéthylammonnium
TEA Ac : Acétate de triéthylammonium contenant 10 % d'acétonitrile.
TCEP : Tris(2-carboxyéthyl)phosphine.

**EXEMPLE 1. Propriétés photophysiques d'un cryptate [TBP-(Eu3+)] libre en présence de sérum:**

Méthode A : Les spectres de fluorescences et les durées de vie sont mesurées sur un Spectrofluorimètre Perkin-Elmer de type LS50.

On prépare une solution mère (concentration de $9.10^{-6}$ M dans du tampon phosphate 100 nM, pH 7) de cryptate [TBP-(Eu3+)]-diamine (purifié par RP-HPLC sur une colonne C-18 avec un gradient linéaire d'acétonitrile dans l'eau contenant 1 % d'acide trifluoroacétique, puis séché sous vide) préparé par réaction de l'éthylènediamine sur le cryptate [(bis-bpy)-(bpy-dimethylester)] décrit dans l'exemple 4, section A de la demande EP 0 321 353. Dans les exemples suivants, ce cryptate [TBP-(Eu3+)]-diamine sera abrégé par K-NH2.

1°) On dilue 200µl de cette solution mère dans 400µl de tampon phosphate 100mM pH7 et on mesure le spectre de fluorescence (td = 0,1ms, tg = 0,4ms, λexcitation = 306nm λémission = 540 à 750nm,-fentes excitation/émission = 10/5, filtre jaune à l'émission) ainsi que la durée de vie t (td = 0,1 à 0,6 ms, tg = 0,4ms, λexcitation = 306nm λémission = 620nm, fentes excitation/émission = 10/5, filtre jaune à l'émission):
On observe que la raie principale ($\lambda_{em}$ = 616nm) présente une durée de vie dans le tampon phosphate de $t_P$ = 0,60ms (Coefficient de Corrélation C.C = 0,999).
2°) On dilue 200µl de cette solution mère de K-NH2 dans un mélange de 200µl de tampon phosphate 100mM pH7 et de 200µl de SVNN et on mesure le spectre et la durée de vie dans les même conditions.

On observe que la raie principale ($\lambda_{em}$ = 616nm) présente une durée de vie dans le tampon phosphate de $t_S$= 0,15ms (C.C = 0,991).
Le facteur d'extinction est donné par l'expression Q = 100-100($t_S/t_P$) soit Q = 100-100(0,15/0,60)= 75 soit 75% d'extinction.

Méthode B :

On prépare une solution mère de cryptate [TBP-(Eu3+)]-diamine dans du tampon phosphate 100mM à une concentration de $1,8.10^{-8}$ M.
On remplit les puits d'une microplaque à fond noir (HTRF 96 puits, Packard) selon le protocole suivant :

*Conditions 1* : On mélange 100µl de solution mère de cryptate avec 100µl de tampon phosphate 100mM pH7 et 100µl de tampon phosphate 100mM pH7 contenant 0,15M NaCl et 0,1% BSA, les mesures sont effectuées en doublet. Ce milieu permet de constituer une référence.
*Conditions 2* : On mélange 100µl de solution mère de cryptate avec 100µl de SVNN et 200µl de tampon phosphate 100mM pH7 contenant 0,15M NaCl et 0,1% BSA (mesure en doublet).

On mesure la fluorescence en temps résolu sur un appareil DISCOVERY (Packard) utilisant une excitation laser à 337nm et une fenêtre d'acquisition de 50µs à 400µs.
Dans le tampon phosphate seul (conditions 1), on observe que l'intensité de l'émission à 620nm est de $1,41.10^5$ ufa (unités de fluorescence arbitraires). Dans les puits adjacents d'une même microplaque, les solutions contenant du sérum (conditions 2) présentent une intensité de l'émission à 620nm de $2,9.10^4$ ufa.
La baisse de l'intensité du signal à 620nm en présence de sérum par rapport à la référence dans le tampon phosphate permet de mettre en évidence le phénomène d'extinction provoqué par le sérum.

$$100 - 100[E_{620}(\text{sérum})/ E_{620}(\text{réf})] = 100 - 100(2,9.10^4 / 1,41.10^5) = 80\%$$

Cette méthode permet une estimation globale de la diminution du signal provenant soit d'une diminution de la durée de vie soit d'une une diminution de l'émission à 620nm.

Elle permet de travailler à des concentrations plus faibles comparativement à la méthode A et elle permet une mesure simultanée de plusieurs échantillons en se plaçant dans des conditions les plus proches possible de celles rencontrées dans un immuno-essai.

**EXEMPLE 2. Synthèse et purification d'un conjugué oligodésoxynucléotide-cryptate [TBP-(Eu3+)]:**

1°) Synthèse d'un oligodésoxynucléotide fonctionnalisé par un bras aminohexyl (AH-ODN1):

Un oligodésoxynucléotide (ODN)de séquence $^{5'\text{-}AH}$C ACG CCA CTA GCT CC-$_{3'}$ modifié en son extrémité 5' par un bras aminohexyl (AH) est synthétisé sur support solide par la méthode dénommée « phosphite-phosphoramidite » en utilisant un synthétiseur d'ADN (Applied Biosystems type 392) selon le protocole du fabriquant. Un nucléotide modifié est introduit en 5' par couplage d'un dérivé N,N-diisopropyl-β-cyanoéthyl-phosphoramidite obtenu à partir de la 5'-O-(4,4'-diméthoxytrityl)-N-4-(6-Trifluoracétamidohexyl)-2'-désoxycytidine préparée par trifluoracétylation de la 5'-O-(4,4'-diméthoxytrityl)-N-4-(6-Aminohexyl)-2'-désoxycytidine comme décrit dans ROGET et al. Nucleic Acids Res., 17, 7643-7650, (1989).

Après synthèse sur un synthétiseur d'ADN (Applied Biosystem 392) en mode "trityl-on" suivant le guide utilisateur correspondant, l'oligonucléotide est traité par l'ammoniaque concentrée (16h à 55°C) et purifié par HPLC sur une colonne LiChrospher$^{R}$ RP-18E 250-10 (10μm)(Merck, Darmstat, D) par un gradient d'acétonitrile dans l'acétate de triéthylammonium 50 mM (tampon A: 5% acétonitrile, tampon B: 50% acétonitrile; débit 5ml/min, gradient de 10% B à 60% B en 20min, isocratique 60% B pendant 5min, puis gradient de 60% B à 100% B en 5min). Selon la méthode décrite dans Oligonucleotide synthesis : A practical approach. Ed M.J. Gait. IRL. Press, Oxford. Les fractions correspondant à un pic majoritaire (temps de rétention supérieur à 20min) sont évaporées. Après évaporation et coévaporation avec de l'eau, l'oligonucléotide partiellement déprotégé ainsi obtenu est détritylé par l'acide acétique à 80% (température ambiante, 30min) puis, après évaporation et coévaporation, l'oligonucléotide complètement déprotégé est repris dans 50μl d'hydrogénocarbonate de triéthylammonium (TEAB) 100mM pH 8 et précipité par 1,5ml de n-butanol. Après centrifugation le surnageant est éliminé et le précipité séché sous vide est repris par 200μl d'eau. Cette solution mère (oligonucléotide dénommé AH-ODN1)présente une absorbtion de 37 $UA_{260}$/ml.

2°) Couplage d'une molécule de cryptate [TBP-(Eu3+)]sur un oligodésoxynucléotide fonctionnalisé par un bras aminohexyl (AH-ODN1):

Une partie aliquote (150μl)de la solution mère de l'oligonucléotide obtenu ci-dessus (5,5 $UA_{260}$ soit environ 39 nmol) est diluée par 150μl d'une solution aqueuse de TEAB 0,1 M pH 7,8 et on ajoute 60 μl d'une solution de cryptate [TBP-(Eu3+)] activé (4 mg/ml) soit 171 nmol (environ 4 équivalents). Le cryptate [TBP-(Eu3+)] activé (N-hydroxysuccinimide/dicydohexytcarbodiimide) est préparée extemporanément à partir de cryptate d'Europium [(bis-bipy)-(bipy-diacide)]lui-même obtenu à partir du cryptate d'Europium [(bis-bipy)-(bipy-dimethylester)] décrit dans l'exemple 4, section A, de la demande EP 0 321 353.

Après 30 min sous agitation, on ajoute 15μl de TEAB 1M pH 8,5 puis évapore sous vide (speed-vac) jusqu'à un volume de 200 μl, on dépose sur une colonne NAP10 (Pharmacia) équilibrée dans un tampon TEAAc 25mM pH7 contenant 10% d'acétonitrile, on élue par le même tampon selon le protocole du fabriquant, la fraction exclue est collectée dans un volume de 1ml, cette fraction est concentrée (speed-vac) jusqu'à un volume de 200μl.

3°) Purification d'un conjugué formé d'un cryptate [TBP-(Eu3+)] et d'un oligodésoxynucléotide fonctionnalisé par un bras aminohexyl (Conjugué KH-ODN1):

Le conjugué KH-ODN1 est analysé par FPLC sur une colonne mono-Q (Pharmacia) en utilisant les conditions suivantes (tampon C : sodium acétate 20mM pH 5 contenant 10% d'acétonitrile. tampon D : sodium acétate 20mM pH 5 lithium chlorure 1 M contenant 10% d'acétonitrile. Gradient : 0 à 2 min isocratique 20% D, 2 min à 30 min gradient de 20% D à 60% D, débit 1 ml/mln).

L'oligonucléotide AH-ODN1 analysé par FPLC dans les conditions ci-dessus présente un temps de rétention Rt = 16,4 min. Dans les mêmes conditions le conjugué KH-ODN1 présente un temps de rétention Rt = 15,4 min.

On injecte ensuite la totalité de la fraction exclue provenant de la colonne NAP10 (200 pl)sur la colonne mono-Q, la fraction correspondant à un temps de rétention de 15 min est collectée, concentrée jusqu'à 300 μl et dessalée sur une colonne NAP10 équilibrée dans un tampon TEAAc 25mM pH7 contenant 10% d'acétonitrile. On élue par le même tampon selon le protocole du fabriquant et la fraction exclue est collectée dans un volume de 1 ml. Cette fraction correspond au conjugué KH-ODN1 pur et est caractérisé par un spectre ultra-violet présentant un maximum à 258 nm

(composante ODN) est un épaulement vers 305 nm (composante cryptate), le rapport des absorbances $A_{260}/A_{305}$ = 4,46 est proche du rapport théorique obtenu en faisant le rapport des absorbances molaires des composants du conjugué pris isolément $\varepsilon_{260}$(ODN)+ $\varepsilon_{260}$(cryptate)/ $\varepsilon_{305}$(cryptate) = (135 000 + 19 000) /30 000 $\simeq$ 5.

La structure du conjugué KH-ODN1 est représentée sur la Figure 1.

4°) Synthèse d'un conjugué cryptate-Oligonucléotide (K-ODN2) :

On répète la synthèse suivant le protocole ci-dessus en construisant la séquence oligonucléotidique GGG GGT TTT TTT TTT ($G_5T_{10}$) à la place de ACG CCA CTA GCT CC.

**EXEMPLE 3. Propriétés photophysiques d'un conjugué Oligonucléotide-cryptate [TBP-(Eu3+)] en présence de sérum:**

<u>Méthode A :</u> Les spectres de fluorescences et les durées de vie sont mesurées sur un Spectrofluorimètre Perkin-Elmer de type LS50.

On utilise la solution mère de conjugué oligonucléotide-cryptate[TBP-(Eu3+)] obtenue dans l'exemple 2 (3°). En considérant la concentration estimée par mesure d'absorbance $\varepsilon_{260}$(conjugué)= $\varepsilon_{260}$(ODN)+ $\varepsilon_{260}$(cryptate)$\simeq$(154 000)] est de $3,5.10^{-6}$ M.

1°) On dilue 200 µl de cette solution mère, soit dans l'eau, soit dans 400 µl de tampon phosphate 100mM pH7 et on mesure le spectre de fluorescence (td = 0,1ms, tg = 0,4ms, λexcitation = 306nm λémission = 540 à 750nm, fentes excitation/émission = 10/5, filtre jaune à l'émission) ainsi que la durée de vie t (td = 0,1 à 0,6 ms, tg=0,4mS, λexcitation = 306nm λémission = 620nm, fentes excitation/émission = 10/5, filtre jaune à l'emission):
Dans l'eau ou dans du tampon phosphate, on observe un profil de spectre différent de celui habituellement observé pour le cryptate [TBP-(Eu3+)]-diamine : la raie principale ($\lambda_{em}$ = 620nm) présente une durée de vie de $t_P$ = 1,1ms (Coefficient de Corrélation C.C = 0,999).
2°) On dilue 200µl de cette solution mère dans un mélange de 200µl de tampon phosphate 100mM pH7 et de 200µl de SVNN et on mesure le spectre et la durée de vie dans les même conditions.

On observe que la raie principale ($\lambda_{em}$ = 620nm) présente une durée de vie dans le tampon phosphate de $t_S$= 1,1ms (C.C = 0,99).

Dans ce cas aucune extinction par diminution de la durée de vie n'est observée.

<u>Méthode B :</u>

On utilise la solution mère de conjugué oligonucléotide-cryptate[TBP-(Eu3+)] obtenue dans l'exemple 2 (3°). Par mesure d'absorbance à 260nm, on estime la concentration à $3,5.10^{-6}$ M. On dilue cette solution mère dans du tampon phosphate 100mM afin d'obtenir une concentration finale de $2.10^{-8}$ M.

On remplit les puits d'une microplaque à fond noir (HTRF 96 puits,Packard) selon le protocole suivant :

*<u>Conditions 1</u>* : On mélange 100µl de solution mère de conjugué K-ODN1 avec 100µl de tampon phosphate 100mM pH7 et 200µl de tampon phosphate 100mM pH7 contenant 0,15M NaCl et 0,1% BSA, les mesures sont effectuées en doublet. Ce milieu permet de constituer une référence.
*<u>Conditions 2</u>* : On mélange 100µl de solution mère de conjugué K-ODN1 avec 100µl de SVNN et 100µl de tampon phosphate 100mM pH7 contenant 0,15M NaCl et 0,1% BSA (mesure en doublet).

On mesure la fluorescence en temps résolu sur un appareil DISCOVERY (Packard) utilisant une excitation laser à 337nm et une fenêtre d'acquisition de 50µs à 400µs.

Dans le tampon phosphate seul (conditions 1), on observe que l'intensité de l'émission à 620nm est de $2,8.10^5$ ufa (unités de fluorescence arbitraire). Dans les puits adjacents d'une même microplaque, les solutions contenant du sérum (conditions 2) présentent un intensité de l'émission à 620nm de $2,8.10^5$ ufa.

Dans ce cas on n'observe pas de baisse de l'intensité du signal à 620nm en présence de sérum par rapport à la référence dans le tampon phosphate. Il n'y a donc pas de phénomène d'extinction provoqué par le sérum.

L'extinction calculée par la relation suivante est de :

$$100 - 100[E_{620}(\text{sérum})/ E_{620}(\text{réf})] = 100 - 100(2{,}8.10^5 / 2{,}8.10^5) = 0\%$$

**EXEMPLE 4. Propriétés photophysiques comparées d'un conjugué Oligonucléotide-cryptate et d'un cryptate [TBP-(Eu3+)] de référence, en présence d'acide urique:**

Cet exemple est utilisé pour comparer l'effet de l'acide urique sur les propriétés photophysiques de différentes molécules contenant un motif cryptate d'europium.

Dans une série de puits d'une microplaque, on pipette des volumes identiques (100µl) soit d'une solution de conjugué marqué au cryptate (environ $2.10^{-8}$ M, voir exemple 3B) que l'on veut évaluer, soit d'une solution de cryptate de référence (environ $2.10^{-8}$ M, voir ex 1B).

Dans chaque série de puits on ajoute dans le premier puits (standard 0) 200µl de tampon phosphate 100mM pH7 contenant 0,15M NaCl et 0,1% BSA et dans les puits suivant 200µl de solutions contenant des concentrations croissantes d'acide urique dans le même tampon (afin d'obtenir par exemple des concentrations finale de 0, 5, 10, 20, 40 et 80 mg/l d'acide urique).

On mesure la fluorescence en temps résolu sur un appareil DISCOVERY (Packard) utilisant une excitation laser à 337nm et une fenêtre d'acquisition de 50µs à 400µs.

Pour chaque série on mesure l'intensité de l'émission à 620nm pour le standard 0 ainsi que pour chaque concentration en acide urique. Pour chaque concentration on évalue le pourcentage d'extinction par la relation suivante:

$$100 - 100[E_{620}(\text{ac. urique}) / E_{620}(\text{standard } 0)]$$

Les résultats sont regroupés dans le tableau I.

TABLEAU 1

| | % d'extinction à 620 nm | |
|---|---|---|
| [Acide urique] en mg/l | K-NH2 | K-G5T10 |
| 0 | 0 | 0 |
| 1,25 | 47 | 8 |
| 2,5 | 66 | 20 |
| 5 | 79 | 28 |
| 10 | 86 | 30 |
| 20 | 88 | 36 |
| 40 | 89 | 40 |
| 80 | 90 | 46 |

On observe que le pourcentage d'extinction du cryptate libre de référence augmente fortement en fonction de la concentration en acide urique. Par contre le pourcentage d'extinction des conjugués cryptate-oligonucléotide est significativement plus bas même pour les fortes concentration en acide urique.

En effet, à la concentration de 5mg/ml le cryptate K-NH2 de référence présente une extinction de 79% alors que dans les mêmes conditions le conjugué cryptate-oligonucléotide K-ODN2 ne présente que 28% d'extinction.

**EXEMPLE 5. Synthèse et purification d'un conjugué cryptate[TBP-(Eu3+)]-aminohexyl-oligonucléotide-Maléimide:**

Synthèse d'un oligonucléotide de séquence $G_5T_{10}$ fonctionnalisé en son extrémité 5' par un cryptate et en son extrémité 3' par un bras portant un groupe réactif maléimide ($^{5'}$K-AH GGG GGT TTT TTT TT $^{MCCAH}$C T-$_{3'}$).

La synthèse est réalisée à partir d'un oligonucléotide portant deux bras aminohexyl dont un est protégé (structure générale MMT-NH-$(CH_2)_6$-($^{5'}ODN_{3'}$)-$(CH_2)_6$-$NH_2$) suivant le schéma ci-après.

MMT-AH GGG GGT TTT TTT TT$^{AH}$CT → $^{5'}$MMT-AH GGG GGT TTT TTT TT$^{MCC\text{-}AH}$CT → $^{5'}$AH GGG GGT TTT TTT

TT$^{MCC-AH}$CT → ($^{5'}$K-AH GGG GGT TTT TTT TT $^{MCC-AH}$C T-$_{3'}$)

Un oligodésoxynucléotide (ODN) de séquence $^{5'}$MMT-AH GGG GGT TTT TTT TT$^{AH}$C T-$_{3'}$ modifié en son extrémité 5' par un bras aminohexyl (AH) sous sa forme protégée par un groupe Monométhoxytrityl (MMT) est synthétisé suivant le procédé suivant :

Par un procédé similaire à l'exemple 1 (1 °), sur une colonne T (1μmol), on couple le dérivé N,N-diisopropyl-β-cyanoethyl-phosphoramidite de la 5'-O-(4,4'-diméthoxytrityl)-N-4-(6-Trifluoracétamidohexyl)-2'-désoxycytidine, ensuite on poursuit la synthèse en construisant la séquence GGG GGT TTT TTT TT, et finalement on effectue le couplage d'un dérivé Monométhoxytrityl-aminohexyl-phosphoramidite (MMT-C6-Aminomodifier. Cruachem) en utilisant l'option « trityl-ON » du synthétiseur. L'oligonucléotide $^{5'}$MMT-AH GGG GGT TTT TTT TT$^{AH}$CT est traité par l'ammoniaque concentrée (16h à 55°C) et purifié par HPLC selon le protocole de l'exemple 1 (1°).

L'oligonucléotide partiellement déprotégé ainsi obtenu est concentré (speed-vac), une partie aliquote (0,24 μmol) est reprise par 100 μl de tampon phosphate 0,1M pH 8 et traité par 5mg de SMCC (15 μmol) dans 100 μl d'acétonitrile, Sigma). Après 40 min sous agitation à température ambiante, le mélange est concentré de moitié (speed-vac)et déssalé sur colonne NAP10 équilibrée dans TEAAc 25mM pH7 5% d'acétonitrile. La fraction exclue (1ml) contenant l'oligonucléotide de structure $^{5'}$MMT-AH GGG GGT TTT TTT TT$^{MCC-AH}$CT est évaporée à sec, le résidu est repris par 1 ml d'acide acétique à 80% après 20 min à température ambiante le mélange est concentré et co-évaporé (speed-vac) par de l'eau puis repris dans 300 μl d'eau. On obtient à ce stade l'oligonucléotide détritylé de structure suivante $^{5'}$AH GGG GGT TTT TTT TT$^{MCC-AH}$CT.

Cet oligonucléotide (0,175 μmol dans 300μl) est dilué par 300μl de TEAB 0,1M pH7 puis on ajoute 450μl (1,27 nmol soit ~ 7 eq.) d'une solution de cryptate [TBP-(Eu3+)] activé (4 mg/ml)comme décrit dans l'exemple 2.

Après 30min sous agitation on évapore sous vide (speed-vac) jusqu'à un volume de 200 μl, on dépose sur une colonne NAP10 (Pharmacia) équilibrée dans un tampon TEAAc 25mM pH7 contenant 10% d'acétonitrile et on élue par le même tampon selon le protocole du fabriquant. La fraction exclue est collectée dans un volume de 1ml et concentrée (speed-vac) jusqu'à un volume de 200μl. La fraction exclue contient principalement l'oligonucléotide marqué de structure ($^{5'}$-**K-AH** GGG GGT TTT TTT TT $^{MCC-AH}$C T-$_{3'}$), cet oligonucléotide est ensuite purifié par injection sur une colonne HR 10/30 remplie de Sephadex G25 éluée par du tampon phosphate 0,1M pH7 avec un débit de 1ml/min. On collecte la fraction éluée entre 8 et 11 min. On obtient ainsi 3 ml d'une solution contenant 17,5 nmol de l'oligonucléotide $^{5'}$-**K-AH** GGG GGT TTT TTT TT $^{MCC-AH}$C T-$_{3'}$ pur directement utilisable pour être couplé sur les fonctions thiol d'une protéine. (rapport $A_{260}/A_{305}$ = 7).

### EXEMPLE 6. Couplage d'un conjugué cryptate[TBP-(Eu3+)]-aminohexyl-oligonucléotide-maléimide sur un anticorps:

Un anticorps est fonctionnalisé par du SPDP (Pierce), et, après réduction par du DTT, l'anticorps activé est purifié sur une colonne HR 10/30 remplie de Sephadex G25 éluée par du tampon phosphate 0,1M pH7 avec un débit de 1ml/min. La fraction contenant l'anticorps activé est combinée avec un conjugué cryptate-oligonucléotide activé par un groupe maléimide $^{5'}$-**K-AH** GGG GGT TTT TTT TT$^{MCC-AH}$C T-$_{3'}$ préparé selon l'exemple 5. Le mélange réactionnel est ensuite purifié sur une colonne Superdex 200 éluée comme ci-dessus, la fraction contenant le conjugué anticorps-oligonucléotide-cryptate est collectée.

### EXEMPLE 7. Couplage d'un conjugué cryptate[TBP-(Eu3+)]-aminohexyl-oligonucléotide-maléimide sur de la streptavidine:

La streptavidine est activée comme dans l'exemple 6 et elle est ensuite marquée à l'aide d'un conjugué cryptate-oligonucléotide activé par un groupe maléimide 5'-**K-AH** GGG GGT TTT TTT TT $^{MCC-AH}$C T-$_{3'}$ préparé selon l'exemple 5.

### EXEMPLE 8. Propriétés photophysiques d'un conjugué protéine-oligonucléotide-cryptate [TBP-(Eu3+)]:

On évalue le pourcentage d'extinction en présence d'acide urique en suivant le protocole de l'exemple 4.

On évalue ainsi un conjugué cryptate[TBP-(Eu3+)]-oligonucléotide-anticorps préparé selon le protocole de l'exemple 6 par rapport à un cryptate[TBP-(Eu3+)]-anticorps de référence préparé par marquage d'un anticorps à l'aide de cryptate (activé par du SMCC) en suivant un protocole classique d'immunochimie.

On évalue également un conjugué cryptate[TBP-(Eu3+)]-oligonucléotide-streptavidine préparé selon l'exemple 7 par rapport à un cryptate[TBP-(Eu3+)]-streptavidine de référence préparé par marquage de streptavidine à l'aide de cryptate (activé par du SMCC) en suivant un protocole classique d'immunochimie.

Les résultats sont regroupés dans le tableau 2.

TABLEAU 2

| | Pourcentage d'extinction du signal du cryptate trisbipyridine à 620 nm | |
|---|---|---|
| [Acide urique] en mg/l | Conjugué anti-prolactine cryptate trisbipyridine | Conjugué anti-prolactine oligonucléotide cryptate trisbipyridine |
| 0 | 0 | 0 |
| 5 | 46 | 2 |
| 10 | 61 | 9 |
| 20 | 71 | 15 |
| 40 | 82 | 20 |
| 80 | 86 | 28 |
| 0 | 0 | 0 |
| 5 | 74 | 10 |
| 10 | 88 | 11 |
| 20 | 94 | 18 |
| 40 | 97 | 23 |
| 80 | 97 | 32 |

On observe que pour les fortes concentrations en acide urique le conjugué cryptate-anticorps de référence présente une extinction de 86% alors que dans les mêmes conditions, le conjugué cryptate-oligonucléotide-anticorps ne présente que 28% d'extinction. Pour une concentration en acide urique voisine entre 5 et 10mg/ml la fluorescence du conjugué de référence est atténuée de 50% alors que le composé de l'invention présente moins de 10% d'extinction.

On observe de façon analogue que le conjugué cryptate-streptavidine de référence présente dans les conditions de forte concentration en acide urique une extinction de 97% alors que le conjugué cryptate-oligonucléotide-streptavidine ne présente que 32 % d'extinction.

**EXEMPLE 9. Couplage d'un conjugué cryptate[TBP-(Eu3+)]-maléimide sur un oligonucléotide-thiol:**

Le cryptate [(bis-bipy)-(bipy-dimethylester)] décrit dans l'exemple 4, section A, de la demande EP 0 321 353 est traité par de l'éthylène diamine et le cryptate-diamine résultant purifié par RP-HPLC est ensuite traité par du SMCC (Pierce) ou du SMP (Pierce) pour introduire un groupe maléimide. On obtient ainsi un conjugué cryptate-maléimide. Ce conjugué cryptate maléimide, purifié sur RP-HPLC est couplé sur un oligonucléotide-thiol (ODN4 ci-dessous).

L'oligonucléotide utilisé possède la structure suivante :

ODN3 : DMT-O$(CH_2)_6$-SS-$(CH_2)_6$-p-d(TTT TTT TTT GGG GG$^{AH}$CG)$_{3'}$.

La fonction thiol est introduite en 5' de l'oligonucléotide sous la forme d'un pont disulfure. Cette fonctionnalisation est introduite à la fin en position 5' de l'oligonucléotide par un phosphoramidite (C6-disulphide phosphoramidite, Cruachem Ltd., Glasgow). Après déprotection ammoniacale et purification (RP-HPLC) l'oligonucléotide est traité par le TCEP (Pierce, Rockford,IL) afin de libérer la fonction thiol.

On obtient ainsi l'oligonucléotide de structure :

ODN4 : HS-$(CH_2)_6$-p-d(TTT TTT TTT GGG GG$^{AH}$CG)$_{3'}$.

On ajoute 15µl d'une solution d'ODN3 à 156 $UA_{260}$/ml à 85µl d'eau, on ajoute 50µl d'une solution de TCEP à 1mg/ml, après 20min à 20°C on dépose sur une colonne NAP 10 (équilibrée dans TEAAc 25mM pH7 5% acétonitrile) on élue la colonne et collecte le volume d'exclusion (1ml contenant environ 9nmol d'ODN4) concentre au speed-vac (jusqu'à environ 100µl) et on ajoute 13 nmol de cryptate-maléimide dans 50µl d'eau. Après une nuit de couplage à 4°C, le mélange est purifié sur NAP10 (élution comme ci-dessus) et le conjugué oligonucléotide-cryptate est élué dans le volume d'exclusion (1 ml). On vérifie par FPLC analytique l'absence de cryptate libre (colonne HR10/30 remplie de Sepharose G25 (Pharmacia), élution par tampon phosphate 10mM pH 7).

On obtient ainsi le conjugué oligonucléotide-cryptate[TBP-$Eu^{3+}$] de structure [bpy.bpy.bpy-$Eu^{3+}$]-S-$(CH_2)_e$-p-d(TTT TTT TTT GGG $GG^{AH}CG)_{3'}$, qui possède près de l'extrémité 3' un bras aminohexyl permettant de lier ce conjugué à une biomolécule.

**EXEMPLE 10. Propriétés photophysiques d'un conjugué Oligonucléotide-cryptate [TBP-$Eu^{3+}$]). obtenu selon l'exemple 9:**

A. Durée de vie :

On effectue la mesure de la durée de vie sur une dilution dans l'eau du conjugué cryptate-oligonucléotide obtenu dans l'exemple 9 en utilisant le protocole de l'exemple 3 (méthode A).

On observe que dans l'eau ou dans le tampon phosphate, la raie principale ($\lambda_{em}$ = 620nm) présente une durée de vie de $t_P$ = 1,33ms (Coefficient de Corrélation C.C = 0,999), cette durée de vie élevée est à rapprocher de la valeur de 1,1ms (exemple 3A) obtenue pour le conjugué cryptate-oligonucléotide dont la synthèse est décrite dans l'exemple 2.

B. Extinction par l'acide urique :

On évalue le pourcentage d'extinction par l'acide urique comme décrit dans l'exemple 4 afin d'obtenir des concentrations finale de 0, 2,5, 5, 10, 20, 40 et 80 mg/l d'acide urique.

On traite de la même façon un échantillon référence de cryptate libre.

Les résultats sont rapportés dans le tableau 3 ci-dessous :

TABLEAU 3

| Acide urique (mg/ml) | Cryptate libre Extinction à 620nm (%) | Conjugué de l'ex. 9 Extinction à 620nm (%) |
|---|---|---|
| 0 | 0 | 0 |
| 2,5 | 81 | 32 |
| 5 | 92 | 38 |
| 10 | 97 | 43 |
| 20 | 98 | 46 |
| 40 | 98 | 47 |
| 80 | 98 | 53 |

On observe que la structure du conjugué cryptate oligonucléotide confère une résistance à l'extinction par l'acide urique du même ordre que ce qui a été observé pour le conjugué de l'exemple 2.

De même la mesure de la durée de vie selon l'exemple 3A montre que la raie principale ($\lambda_{em}$ = 620nm) présente une durée de vie dans le tampon phosphate de $t_S$= 1,1 ms (C.C = 0,99).

Par conséquent, la façon de créer la liaison covalente entre l'unité cryptate et l'oligonucléotide n'influe pas de façon sensible sur les propriétés photophysiques des conjugués.

**EXEMPLE 11. Couplage d'un conjugué cryptate[bis-diethoxybpy.bpy-(Eu3+)]-maléimide sur un oligonucléotide-thiol:**

Dans cet exemple on utilise un cryptate formé de deux unités 4,4'-diéthoxy-2,2'-bipyridines et d'une unité 4,4'-di-methylcarboxylate-2,2'-bipyridine. La synthèse de ce cryptate est faite selon le procédé décrit dans la demande EP 0 321 353, par condensation, en présence de carbonate de sodium dans l'acétonitrile à reflux, de 2 équivalents de 6,6'-dibromométhyl-4,4-diéthoxy-2,2'-bipyddine et 1 équivalent de dérivé 6,6'-di-aminométhyl-4,4'-diméthylcarboxylate-2,2'-bipyridine. On obtient ainsi le cryptate [bis-diéthoxybpy. di$COOCH_3$bpy]NaBr. Ce cryptate de sodium est ensuite converti en cryptate d'europium [bis-diéthoxybpy.di$COOCH_3$bpy-$Eu^{3+}$] par $EuCl_3.6H_2O$ dans le méthanol à reflux. Ce cryptate-diméthylester d'europium est ensuite traité par de l'éthylène diamine (4h à 20°C), le cryptate-diamine d'europium [bis-diéthoxybpy.(di-$NH_2(CH_2)_2$-NHCO-bpy)-$Eu^{3+}$] résultant est purifié par RP-HPLC.

Ce cryptate servira de cryptate de référence et sera appelé K'NH2 dans l'exemple 12 ci-dessous. Il est ensuite traité par du SMP (Pierce) pour introduire un groupe maléimide. On obtient ainsi un conjugué cryptate [bis-diéthoxy-bpy.bipy-$Eu^{3+}$]-maléimide. Ce conjugué cryptate-maléimide, purifié sur RP-HPLC est couplé selon le protocole de l'exemple 9 sur l'oligonucléotide-thiol ODN4 décrit dans cet exemple. On obtient ainsi un conjugué oligonucléotide-[bis-dié-

thoxybpy.bpy-($Eu^{3+}$)]. Le spectre UV de ce conjugué présente un maximum vers 260nm correspondant à l'oligonucléotide et 2 épaulements vers 305 nm et 337nm correspondant à la partie cryptate.

**EXEMPLE 12. Propriétés photophysiques du conjugué oligonucléotide-cryptate[bis-diethoxybpy.bpy-(Eu3+)] obtenu selon l'exemple 11 :**

On effectue les mesures de durée de vie du conjugué oligonucléotide-cryptate[bis-diethoxybpy.bpy-(Eu3+)] (exemple 11) dans le tampon phosphate, selon le protocole décrit dans l'exemple et en utilisant comme cryptate de référence le composé K'NH2 décrit dans l'exemple 11.

Les résultats sont rapportés dans le tableau 4 ci-dessous :

TABLEAU 4

| Milieu | Conjugué oligonucléotide-cryptate[bis-diethoxybpy.bpy-(Eu3+)] Durée de vie (ms) | K'NH2 Durée de vie (ms) |
|---|---|---|
| Phosphate | 0,8 | 0,6 |
| Phosphate + sérum | 0,8 | 0,2 |

On observe donc que le K'NH2 de référence mis en présence de sérum montre un phénomène d'extinction qui se traduit par une baisse significative de la durée de vie en comparaison avec la valeur observée dans le phosphate seul.

On observe que le conjugué oligonucléotide-cryptate[bis-diethoxybpy.bpy-(Eu3+)] n'est pas affecté par le sérum.

Cet exemple montre que l'effet protecteur de la partie oligonucléotide est indépendante de la structure du cryptate.

**Revendications**

1. Procédé de réduction de l'extinction de fluorescence due au milieu de mesure dans un dosage par fluorescence d'un analyte mettant en oeuvre au moins un marqueur fluorescent, **caractérisé en ce qu'**on introduit dans le milieu de mesure un conjugué fluorescent comprenant un oligonucléotide lié à un cryptate de terre rare et **en ce que** ledit conjugué est lié de manière covalente à une molécule biologique ayant un rôle de reconnaissance et pouvant se lier à un partenaire, ladite molécule biologique étant l'un des membres d'un couple de molécules capables de se lier spécifiquement entre elles, sélectionnée parmi les couples suivants : couple ligand / récepteur, couple antigène / anticorps, couple biotine / avidine, couple acide nucléique /acide nucléique, sous réserve que lorsque la molécule biologique est un acide nucléique, ledit oligonucléotide est choisi parmi les oligonucléotides suivants : -$^{(AH)}$C ACG CCA CTA GCT CC-, -$^{(AH)}$C GGG GGT TTT TTT TTT-, -$^{(AH)}$GGG GGT TTT TTT TT$^{(MCC-AH)}$ CT-, -TTT TTT TTT GGG GG$^{(AH)}$CG-.

2. Procédé selon la revendication 1, **caractérisé en ce que** l'oligonucléotide est constitué d'un enchaînement d'unités ribonucléotides ou désoxyribonucléotides liées entre elles par des liaisons de type phosphodiester.

3. Procédé selon l'une des revendications 1 ou 2, **caractérisé en ce que** l'oligonucléotide est constitué d'un enchaînement d'unités ribonucléotides ou désoxyribonucléotides ou d'unités analogues de nucléotide modifiées sur le sucre ou sur la base, liées entre elles par des liaisons internucléotidiques naturelles de type phosphodiester, une partie des liaisons internucléotidiques étant éventuellement remplacée par des liaisons phosphonate, phosphoramide ou phosphorothioate.

4. Procédé selon l'une des revendications 1 ou 2, **caractérisé en ce que** l'oligonucléotide est constitué d'un enchaînement comprenant à la fois des unités ribonucléotides ou désoxyribonucléotides liées entre elles par des liaisons de type phosphodiester et des unités analogues de nucléosides liées entre elles par des liaisons amide.

5. Procédé selon l'une quelconque des revendications 1 à 4, **caractérisé en ce que** l'oligonucléotide est constitué d'unités ribonucléotides ou désoxyribonucléotides, dont l'une au moins peut comporter un groupe fonctionnel introduit ou généré sur ladite unité ou un groupe fonctionnel introduit à l'aide d'un bras d'espacement lié au groupement phosphate terminal en position 3' ou 5'.

6. Procédé selon la revendication 5, **caractérisé en ce que** ladite unité est l'unité 5' terminale ou 3' terminale.

7. Procédé selon l'une quelconque des revendications 1 à 6, **caractérisé en ce que** l'oligonucléotide comprend un enchaînement de 5 à 50 nucléotides ou un enchaînement de 5 à 50 nucléotides et analogues de nucléotides, ou de nucléosides.

8. Procédé selon l'une quelconque des revendications 1 à 6, **caractérisé en ce que** l'oligonucléotide est constitué d'un enchaînement d'unités ribonucléotides ou désoxyribonucléotides liées entre elles par des liaisons de type phosphodiester et d'unités analogues de nucléosides liées entre elles par des liaisons amide, ledit oligonucléotide comprenant au moins 5 liaisons internucléotidiques de type phosphodiester à l'extrémité destinée à être liée au cryptate.

9. Procédé selon l'une quelconque des revendications 1 à 8, **caractérisé en ce que** le cryptate de terre rare est lié de manière covalente à l'oligonucléotide soit directement, soit par l'intermédiaire d'un bras d'espacement.

10. Procédé selon l'une quelconque des revendications 1 à 9, **caractérisé en ce que** ledit cryptate de terre rare est constitué d'au moins un sel de terre rare complexé par un composé macropolycyclique de formule

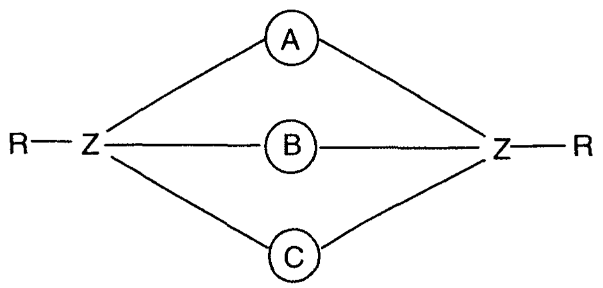

I

dans laquelle Z est un atome ayant 3 ou 4 valences, R est rien ou représente l'hydrogène, le groupe hydroxy, un groupe amino ou un radical hydrocarboné, les radicaux bivalents Ⓐ, Ⓑ et Ⓒ, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ et Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

11. Procédé selon la revendication 10, **caractérisé en ce que** le cryptate de terre rare est constitué d'un sel de terre rare complexé par l'un des composés macrocycliques ou macropolycycliques ci-après :

(22)phénanthroline ; (22)phénanthroline amide ; (22)anthracène ; (22)anthracène amide ; (22)bi-isoquinoléine ; (22)biphényl-bis-pyridine ; (22)bipyridine ; (22)bi-pyridine amide ; les macropolycycles tris-bipyridine, tris-phénanthroline, phénanthroline-bis-bipyridine, bi-isoquinoléine-bis-bipyridine, bis-bipyridine diphénylbipyridine ; un composé macropolycyclique comprenant un motif moléculaire choisi parmi les bipyrazines, les bipyrimidines

et les hétérocycles azotés comportant des groupements N-oxydes....

**12.** Procédé selon l'une quelconque des revendications 1 à 9, **caractérisé en ce que** le cryptate de terre rare est constitué d'au moins un sel de terre rare complexé par un composé macropolycyclique répondant à l'une des formules II ou III ci-après : 1

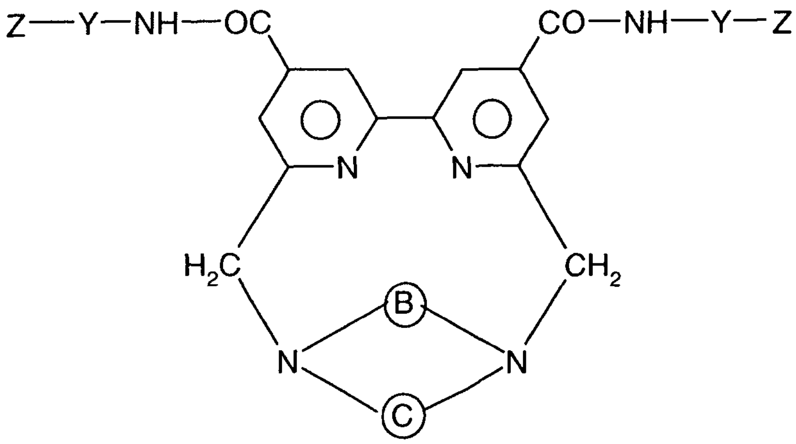

II

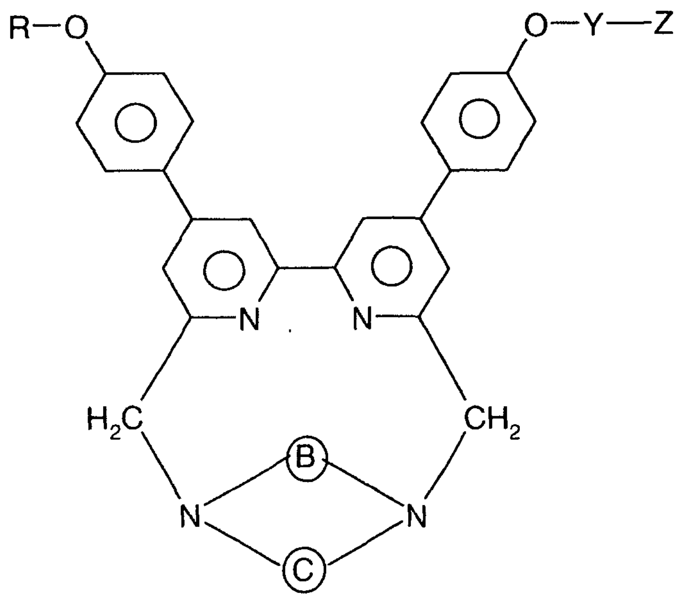

III

dans lesquels :

- le cycle de formule

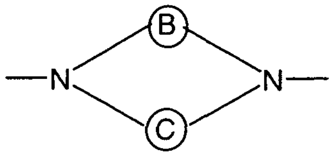

est l'un des cycles suivants :

1)

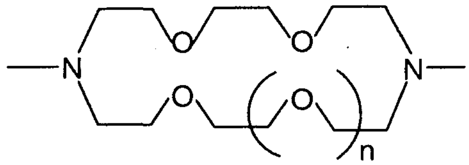

n = 0 ou 1
macrocycle $[N_2O_4]$ ou cycle (22)
macrocycle $[N_2O_3]$ ou cycle (21)

2)

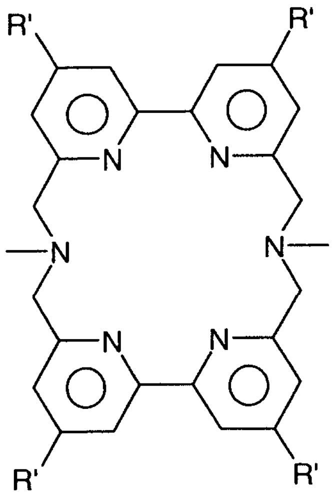

macrocycle bis-bipyridine

- Y est un groupe ou un bras d'espacement qui est constitué par un radical organique bivalent, choisi parmi les groupes alkylène linéaires ou ramifiés en $C_1$ à $C_{20}$ contenant éventuellement une ou plusieurs doubles liaisons et/ou éventuellement contenant un ou plusieurs hétéroatomes tels que l'oxygène, l'azote, le soufre, le phosphore ou un ou plusieurs groupe(s) carbamoyle ou carboxamido ; parmi les groupes cycloalkylène en $C_5$ à $C_8$ ou parmi les groupes arylène en $C_6$ à $C_{14}$, lesdits groupes alkylène, cycloalkylène ou arylène étant éventuellement substitués par des groupes alkyle, aryle ou sulfonate ;
- Z est un groupe fonctionnel susceptible de se lier de façon covalente avec une substance biologique ;
- R est un groupe méthyle ou représente le groupe -Y-Z ;
- R' est l'hydrogène ou un groupe -COOR" dans lequel R" est un groupe alkyle en $C_1$ à $C_{10}$ et représente de préférence le groupe méthyle, éthyle ou tertiobutyle ou bien R' est un groupe -CO-NH-Y-Z.

**13.** Procédé selon l'une quelconque des revendications 1 à 12, **caractérisé en ce que** le cryptate de terre rare est lié à l'oligonucléotide par l'intermédiaire d'un bras d'espacement constitué par un radical organique bivalent, choisi parmi les groupes alkylène linéaires ou ramifiés en $C_1$-$C_{20}$, contenant éventuellement une ou plusieurs doubles liaisons ou triples liaisons et/ou contenant éventuellement un ou plusieurs hétéroatomes, tels que l'oxygène, l'azote, le soufre, le phosphore ou un ou plusieurs groupe(s) carbamoyle ou carboxamido ; les groupes cycloalkylène en $C_5$-$C_8$ et les groupes arylène en $C_6$-$C_{14}$, lesdits groupes alkylène, cycloalkylène ou arylène étant éventuellement substitués par des groupes alkyle, aryle ou sulfonate.

**14.** Procédé selon la revendication 13, **caractérisé en ce que** le bras d'espacement est choisi parmi les groupes :

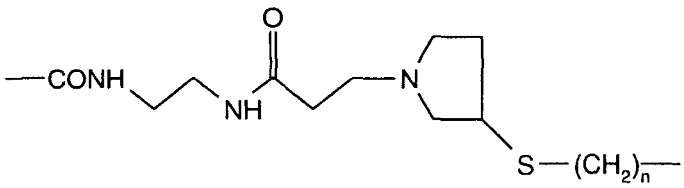

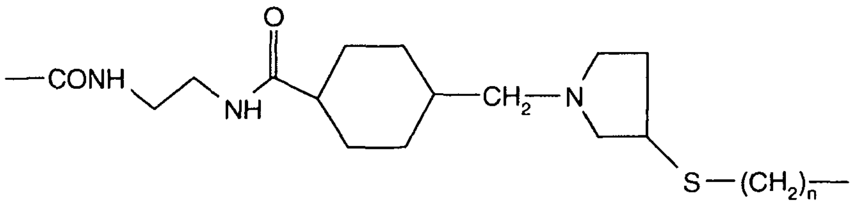

dans lesquelles n = 2 à 6, et $-CONH-(CH_2)_6-$, la liaison via le groupe -CONH ayant lieu au niveau du cryptate.

**15.** Procédé selon l'une quelconque des revendications 1 à 14, **caractérisé en ce que** le cryptate de terre rare est un cryptate d'europium.

**16.** Procédé selon la revendication 15, **caractérisé en ce que** le cryptate de terre rare est le cryptate d'europium Eu trisbipyridine ou Eu [bis-diéthoxybipyridine.bipyridine].

**17.** Procédé selon l'une quelconque des revendications 1 à 16, **caractérisé en ce que** le conjugué fluorescent est utilisé comme seul marqueur ou comme l'un des marqueurs fluorescents dans le dosage.

**18.** Procédé selon l'une quelconque des revendications 1 à 17, **caractérisé en ce qu'**en plus dudit conjugué fluorescent, on met en oeuvre dans le dosage un marqueur fluorescent comprenant un composé fluorescent accepteur.

**19.** Conjugué fluorescent, **caractérisé en ce qu'**il comprend :

(i) un cryptate de terre rare,
(ii) un oligonucléotide,
(iii) une molécule biologique ayant un rôle de reconnaissance et pouvant se lier à un partenaire, ladite molécule biologique étant l'un des membres d'un couple de molécules capables de se lier spécifiquement entre elles, sélectionnée parmi les couples suivants : couple ligand / récepteur, couple antigène / anticorps, couple biotine / avidine, couple acide nucléique /acide nucléique, sous réserve que lorsque la molécule biologique est un acide nucléique, ledit oligonucléotide est choisi parmi les oligonucléotides suivants : -$^{(AH)}$C ACG CCA CTA GCT CC-, -$^{(AH)}$C GGG GGT TTT TTT TTT-, -$^{(AH)}$GGG GGT TTT TTT TT$^{5MCC-AH)}$ CT-, -TTT TTT TTT GGG GG$^{(AH)}$CG-.

(i), (ii) et (iii) étant liés par des liaisons covalentes.

**20.** Conjugué selon la revendication 19, **caractérisé en ce que** l'oligonucléotide est constitué d'un enchaînement d'unités ribonucléotides ou désoxyribonucléotides liées entre elles par des liaisons de type phosphodiester.

**21.** Conjugué selon l'une des revendications 19 ou 20, **caractérisé en ce que** l'oligonucléotide est constitué d'un enchaînement d'unités ribonucléotides ou désoxyribonucléotides ou d'unités analogues de nucléotide modifiées sur le sucre ou sur la base, liées entre elles par des liaisons internucléotidiques naturelles de type phosphodiester, une partie des liaisons internucléotidiques étant éventuellement remplacée par des liaisons phosphonate, phosphoramide ou phosphorothioate.

**22.** Conjugué selon l'une quelconque des revendications 19 à 20, **caractérisé en ce que** l'oligonucléotide est constitué d'un enchaînement comprenant à la fois des unités ribonucléotides ou désoxyribonucléotides liées entre elles par des liaisons de type phosphodiester et des unités analogues de nucléosides liées entre elles par des liaisons amide.

**23.** Conjugué selon l'une quelconque des revendications 19 à 22, **caractérisé en ce que** l'oligonucléotide est constitué d'unités ribonucléotides ou désoxyribonucléotides, dont l'une au moins peut comporter un groupe fonctionnel introduit ou généré sur ladite unité ou un groupe fonctionnel introduit à l'aide d'un bras d'espacement lié au groupement phosphate terminal en position 3' ou 5'.

**24.** Conjugué selon la revendication 23, **caractérisé en ce que** ladite unité est l'unité 5' terminale ou 3' terminale.

**25.** Conjugué selon l'une quelconque des revendications 19 à 24, **caractérisé en ce que** l'oligonucléotide comprend un enchaînement de 5 à 50 nucléotides ou un enchaînement de 5 à 50 nucléotides et analogues de nucléotides, ou de nucléosides.

**26.** Conjugué selon l'une quelconque des revendications 19 à 25, **caractérisé en ce que** l'oligonucléotide est constitué d'un enchaînement d'unités ribonucléotides ou désoxyribonucléotides liées entre elles par des liaisons de type phosphodiester et d'unités analogues de nucléosides liées entre elles par des liaisons amide, ledit oligonuctéotide comprenant au moins 5 liaisons internucléotidiques de type phosphodiester à l'extrémité destinée à être liée au cryptate.

**27.** Conjugué selon l'une quelconque des revendications 19 à 26, **caractérisé en ce que** le cryptate de terre rare est lié de manière covalente à l'oligonucléotide soit directement, soit par l'intermédiaire d'un bras d'espacement.

**28.** Conjugué selon l'une quelconque des revendications 19 à 27, **caractérisé en ce que** ledit cryptate de terre rare est constitué d'au moins un sel de terre rare complexé par un composé macropolycyclique de formule :

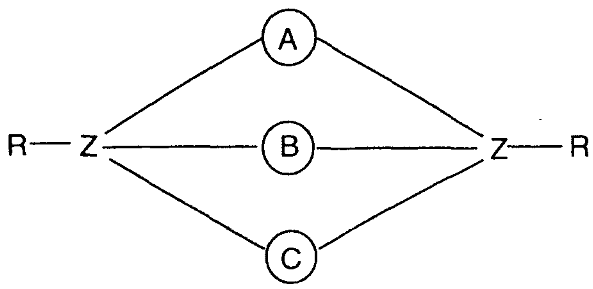

I

dans laquelle Z est un atome ayant 3 ou 4 valences, R est rien ou représente l'hydrogène, le groupe hydroxy, un groupe amino ou un radical hydrocarboné, les radicaux bivalents Ⓐ, Ⓑ et Ⓒ, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ et Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

**29.** Conjugué selon l'une quelconque des revendications 19 à 28, **caractérisé en ce que** le cryptate de terre rare est constitué d'un sel de terre rare complexé par l'un des composés macrocycliques ou macropolycycliques ci-après : (22)phénanthroline ; (22)phénanthroline amide ; (22)anthracène ; (22)anthracène amide ; (22)bi-isoquinoléine ; (22) biphényl-bis-pyridine ; (22)bipyridine ; (22)bi-pyridine amide ; les macropolycycles tris-bipyridine, tris-phénanthroline, phénanthroline-bis-bipyridine, bi-isoquinoléine-bis-bipyridine, bis-bipyridine diphénylbipyridine ; un composé macropolycyclique comprenant un motif moléculaire choisi parmi les bipyrazines, les bipyrimidines et les hétérocycles azotés comportant des groupements N-oxydes.

**30.** Conjugué selon l'une quelconque des revendications 19 à 29, **caractérisé en ce que** le cryptate de terre rare est constitué d'au moins un sel de terre rare complexé par un composé macropolycyclique répondant à l'une des formules II ou III ci-après :

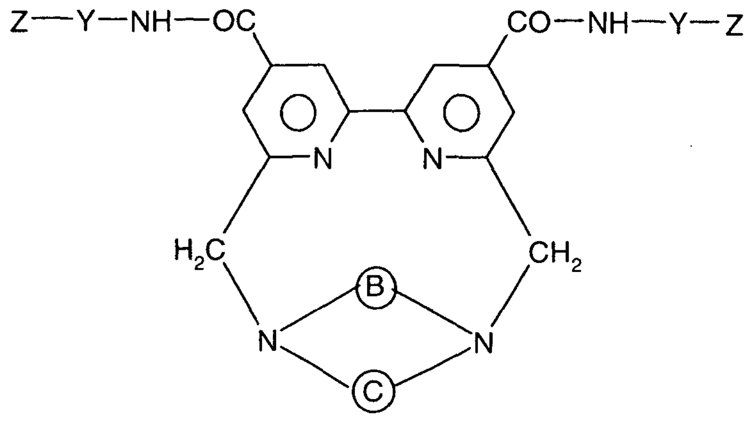

II

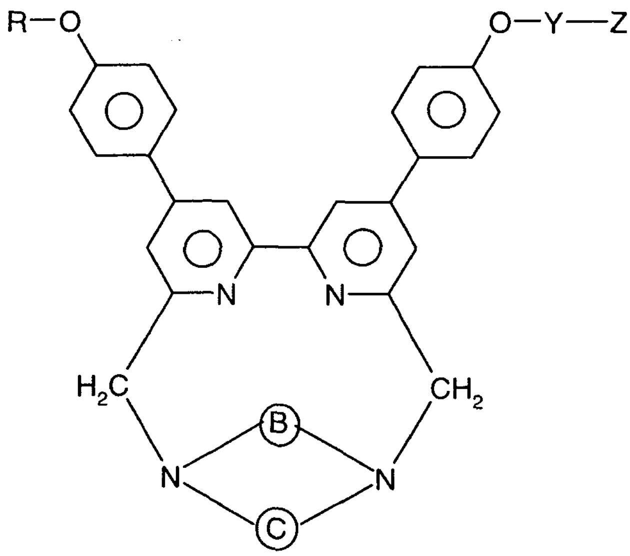

III

dans lesquels :

le cycle de formule

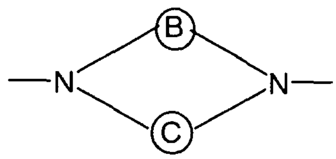

est l'un des cycles suivants :

1)

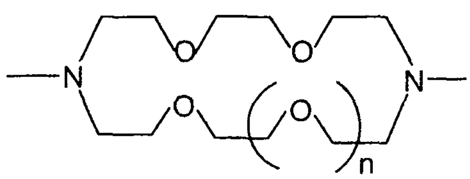

n = 0 ou 1
macrocycle [$N_2O_4$] ou cycle (22)
macrocycle [$N_2O_3$] ou cycle (21)

2)

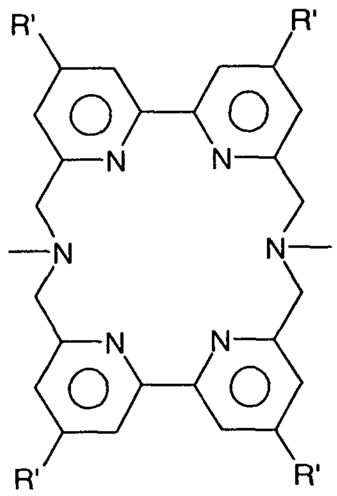

macrocycle bis-bipyridine

Y est un groupe ou un bras d'espacement qui est constitué par un radical organique bivalent, choisi parmi les groupes alkylène linéaires ou ramifiés en $C_1$ à $C_{20}$ contenant éventuellement une ou plusieurs doubles liaisons et/ou éventuellement contenant par un ou plusieurs hétéroatomes tels que l'oxygène, l'azote, le soufre, le phosphore ou un ou plusieurs groupe(s) carbamoyle ou carboxamido ; parmi les groupes cycloalkylène en $C_5$ à $C_8$ ou parmi les groupes arylène en $C_6$ à $C_{14}$, lesdits groupes alkylène, cycloalkylène ou arylène étant éventuellement substitués par des groupes alkyle, aryle ou sulfonate ;
Z est un groupe fonctionnel susceptible de se lier de façon covalente avec une substance biologique ;
R est un groupe méthyle ou représente le groupe -Y-Z ;
R' est l'hydrogène ou un groupe -COOR" dans lequel R" est un groupe alkyle en $C_1$ à $C_{10}$ et représente de préférence le groupe méthyle, éthyle ou tertiobutyle ou bien R' est un groupe -CO-NH-Y-Z.

**31.** Conjugué selon l'une quelconque des revendications 19 à 30, **caractérisé en ce que** le cryptate de terre rare est lié à l'oligonucléotide par l'intermédiaire d'un bras d'espacement constitué par un radical organique bivalent, choisi parmi les groupes alkylène linéaires ou ramifiés en $C_1$-$C_{20}$, contenant éventuellement une ou plusieurs doubles liaisons ou triples liaisons et/ou contenant éventuellement un ou plusieurs hétéroatomes, tels que l'oxygène, l'azote, le soufre, le phosphore ou un ou plusieurs groupe(s) carbamoyle ou carboxamido ; les groupes cycloalkylène en $C_5$-$C_8$ et les groupes arylène en $C_6$-$C_{14}$, lesdits groupes alkylène, cycloalkylène ou arylène étant éventuellement substitués par des groupes alkyle, aryle ou sulfonate.

**32.** Conjugué selon l'une quelconque des revendications 19 à 31, **caractérisé en ce que** le bras d'espacement est choisi parmi les groupes :

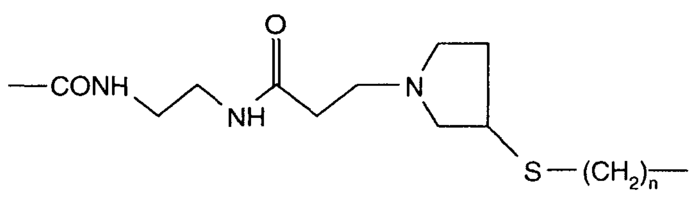

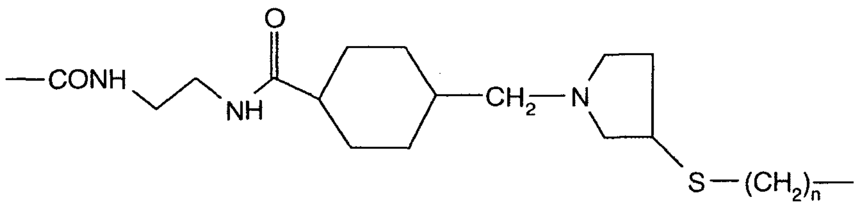

dans lesquelles n = 2 à 6, et $-CONH-(CH_2)_6-$, la liaison via le groupe -CONH ayant lieu au niveau du cryptate.

**33.** Conjugué selon l'une quelconque des revendications 19 à 32, **caractérisé en ce que** le cryptate de terre rare est un cryptate d'europium.

**34.** Conjugué selon l'une quelconque des revendications 19 à 33, **caractérisé en ce que** le cryptate de terre rare est le cryptate d'europium Eu trisbipyridine ou Eu [bis-diéthoxybipyridine.bipyridine].

## Claims

**1.** Process for reducing the extinction of fluorescence due to the measurement medium in one fluorescence assay of an analyte using at least one fluorescent marker, **characterized in that** a fluorescent conjugate comprising an oligonucleotide bonded to a rare-earth cryptate is introduced into the measurement medium, and **in that** said conjugate is covalently bonded to a biological molecule having a recognition role and which can bind to a partner, said biological molecule being one member of a pair of molecules capable of binding specifically to one another, selected amongst the following pairs: ligand/receptor pair, antigene/antibody pair, biotine/avidine pair, nucleic acid/nucleic acid pair, on the condition that when the biological molecule is a nucleic acid, said oligonucleotide is selected from the following oligonucleotides: -$^{(AH)}$C ACG CCA CTA GCT CC-, -$^{(AH)}$C GGG GGT TTT TTT TTT-, -$^{(AH)}$GGG GGT TTT TTT TT$^{(MCC-AH)}$CT-, -TTT TTT TTT GGG GG$^{(AH)}$CG-.

**2.** The process according to claim 1, **characterized in that** the oligonucleotide consists of a chain of ribonucleotide or deoxyribonucleotide units bonded to one another via phosphodiester-type bonds.

**3.** The process according to claim 1 or 2, **characterized in that** the oligonucleotide consists of a chain of ribonucleotide or deoxyribonucleotide units or of analogous units of nucleotides modified on the sugar or on the base and bonded to one another via natural phosphodiester-type internucleotide bonds, some of the internucleotide bonds optionally being replaced with phosphonate, phosphoramide or phosphorothioate bonds.

**4.** The process according to claim 1 or 2, **characterized in that** the oligonucleotide consists of a chain comprising both ribonucleotide or deoxyribonucleotide units bonded to one another via phosphodiester-type bonds and analogous units of nucleosides bonded to one another via amide bonds.

**5.** The process according to any one of claims 1 to 4, **characterized in that** the oligonucleotide consists of ribonucleotide or deoxyribonucleotide units, at least one of which may comprise a functional group introduced onto or generated on said unit, or a functional group introduced by using a spacer arm bonded to the terminal phosphate group in the 3' or 5' position.

**6.** The process according to claim 5, **characterized in that** said unit is the 5' terminal unit or 3' terminal unit.

**7.** The process according to any one of claims 1 to 6, **characterized in that** the oligonucleotide comprises a chain of 5 to 50 nucleotides or a chain of 5 to 50 nucleotides and nucleotide analogs or nucleoside analogs.

**8.** The process according to any one of claims 1 to 6, **characterized in that** the oligonucleotide consists of a chain of ribonucleotide or deoxyribonucleotide units bonded to one another via phosphodiester-type bonds and of analogous nucleoside units bonded to one another via amide bonds, said oligonucleotide comprising at least 5 phosphodiester-type internucleotide bonds at the end intended to be bonded to the cryptate.

**9.** The process according to any one of claims 1 to 8, **characterized in that** the rare-earth metal cryptate is bonded

covalently to the oligonucleotide either directly or via a spacer arm.

**10.** The process according to any one of claims 1 to 9, **characterized in that** said rare-earth metal cryptate consists of at least one rare-earth metal salt complexed with a macropolycyclic compound of formula

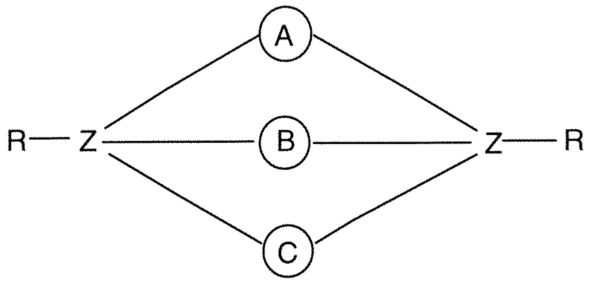

I

in which Z is an atom with 3 or 4 valencies, R is nothing or represents hydrogen, a hydroxy group, an amino group or a hydrocarbon-based radical, the divalent radicalsⒶ , Ⓑ and Ⓒ, are, independently of each other, hydrocarbon-based chains which optionally contain one or more hetero atoms and are optionally interrupted with a heteromacrocycle, at least one of the radicals Ⓐ, Ⓑ and Ⓒ, also comprising at least one molecular unit or consisting essentially of a molecular unit, said molecular unit having a triplet energy which is greater than that of the emission level of the complexed rare-earth metal ion.

**11.** The process according to claim 10, **characterized in that** the rare-earth metal cryptate consists of a rare-earth metal salt complexed with one of the macrocyclic or macropolycyclic compounds below:

(22)phenanthroline; (22)phenanthroline amide; (22)anthracene; (22)anthracene amide; (22)bi-isoquinoline; (22) biphenyl-bis-pyridine; (22)bipyridine; (22)bi-pyridine amide; the macropolycycles tris-bipyridine, trisphenanthroline, phenanthroline-bis-bipyridine, bi-isoquinoline-bis-bipyridine, bis-bipyridine diphenylbipyridine; a macropolycyclic compound comprising a molecular unit chosen from bipyrazines, bipyrimidines and nitrogen-containing heterocycles comprising N-oxide groups.

**12.** The process according to any of claims 1 to 9, **characterized in that** the rare-earth metal cryptate consists of at least one rare-earth metal salt complexed with a macropolycyclic compound corresponding to one of the formulae II or III below:

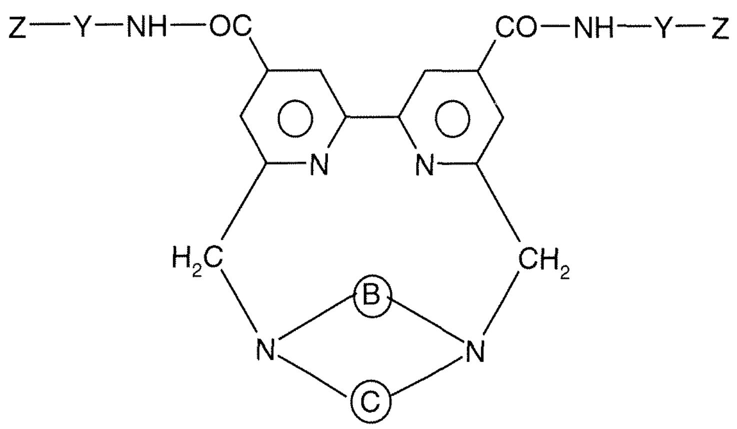

II

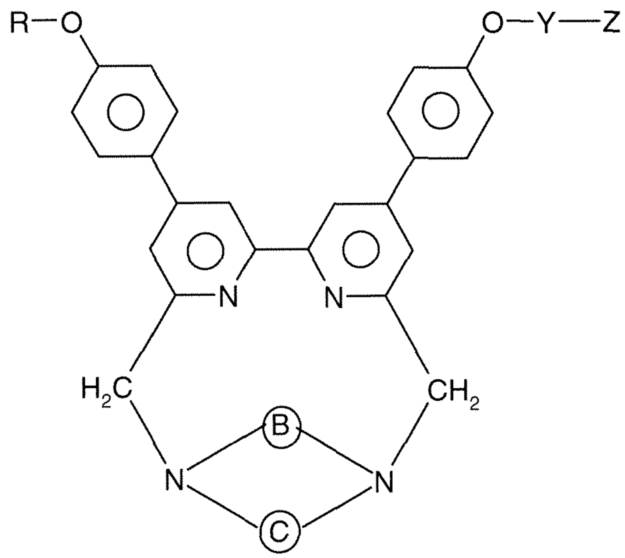

III

in which:

- the ring of formula

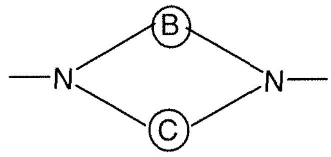

is one of the following rings:

1)

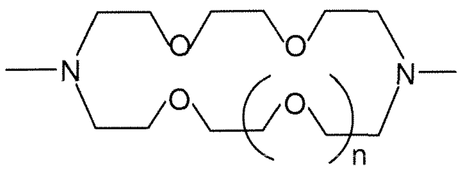

n = 0 or 1

$[N_2O_4]$ macrocycle or cycle (22)

$[N_2O_3]$ macrocycle or cycle (21)

2)

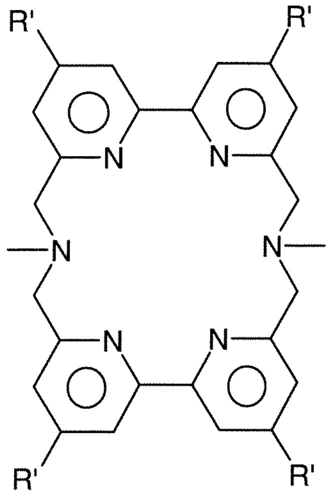

bis-bipyridine macrocylcle

- Y is a spacer group or spacer arm which consists of a divalent organic radical, chosen from linear or branched $C_1$ to $C_{20}$ alkylene groups optionally containing one or more double bonds and/or optionally containing one or more heteroatoms such as oxygen, nitrogen, sulfur or phosphorus or one or more carbamoyl or carboxamido group(s); chosen from $C_5$ to $C_8$ cycloalkylene groups or chosen from $C_6$ to $C_{14}$ arylene groups, said alkylene, cycloalkylene or arylene groups being optionally substituted with alkyl, aryl or sulfonate groups;
- Z is a functional group capable of bonding covalently to a biological substance;
- R is a methyl group or represents the group -Y-Z;
- R' is hydrogen or a group -COOR" in which R" is a $C_1$ to $C_{10}$ alkyl group and preferably represents a methyl, ethyl or tert-butyl group, or alternatively R' is a group -CO-NH-Y-Z.

**13.** The process according to any one of claims 1 to 12, **characterized in that** the rare-earth metal cryptate is bonded to the oligonucleotide via a spacer arm consisting of a divalent organic radical chosen from $C_1$-$C_{20}$ linear or branched alkylene groups optionally containing one or more double bonds or triple bonds and/or optionally containing one or more hetero atoms, such as oxygen, nitrogen, sulfur, phosphorus or one or more carbamoyl or carboxamido group (s); $C_5$-$C_8$ cycloalkylene groups and $C_6$-$C_{14}$ arylene groups, said alkylene, cycloalkylene or arylene groups being optionally substituted with alkyl, aryl or sulfonate groups.

**14.** The process according to claim 13, **characterized in that** the spacer arm is chosen from the groups:

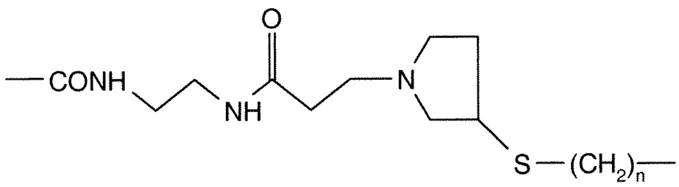

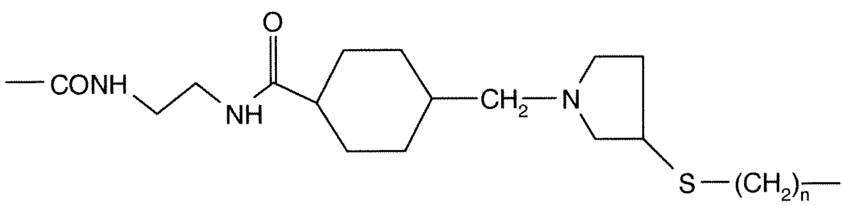

in which n = 2 to 6, and -CONH-$(CH_2)_6$-, the attachment via the group -CONH taking place on the cryptate.

**15.** The process according to any one of claims 1 to 14, **characterized in that** the rare-earth metal cryptate is a europium

cryptate.

**16.** The process according to claim 15, **characterized in that** the rare-earth metal cryptate is the europium cryptate Eu trisbipyridine or Eu [bis-diethoxybipyridine.bipyridine].

**17.** The process according to any one of claims 1 to 16, **characterized in that** the fluorescent conjugate is used as the only label or as one of the fluorescent labels in the assay.

**18.** The process according to any of claims 1 to 17, **characterized in that**, in addition to said fluorescent conjugate, a fluorescent label comprising an acceptor fluorescent compound is used in the assay.

**19.** Conjugate, comprising:

(i) a rare-earth cryptate;
(ii) an oligonucleotide;
(iii) a biological molecule having a recognition role and which can bind to a partner, said biological molecule being one of a pair of molecules capable of binding specifically to one another, selected amongst the following pairs: ligand/receptor pair, antigene/antibody pair, biotine/avidine pair, nucleic acid/nucleic acid pair, on the condition that when the biological molecule is a nucleic acid, said oligonucleotide is selected from the following oligonucleotides: -$^{(AH)}$C ACG CCA CTA GCT CC-, -$^{(AH)}$C GGG GGT TTT TTT TTT-, -$^{(AH)}$GGG GGT TTT TTT TT$^{(MCC-AH)}$CT-, -TTT TTT TTT GGG GG$^{(AH)}$CG-.

(i), (ii) and (iii) being linked by covalent bonds.

**20.** Conjugate according to claim 19, **characterized in that** the oligonucleotide consists of a chain of ribonucleotide units or deoxyribonucleotide units bonded to one another via phosphodiester-type bonds.

**21.** The conjugate according to any one of claims 19 or 20, wherein the oligonucleotide consists of a chain of ribonucleotide or deoxyribonucleotide units or of analogous units of nucleotides modified on the sugar or on the base and bonded to one another via natural phosphodiester-type internucleotide bonds, some of the internucleotide bonds optionally being replaced with phosphonate, phosphoramide or phosphorothioate bonds.

**22.** The conjugate according to any one of claims 19 to 20, **characterized in that** the oligonucleotide consists of a chain comprising both ribonucleotide or deoxyribonucleotide units bonded to one another via phosphodiester-type bonds and analogous units of nucleosides bonded to one another via amide bonds.

**23.** The conjugate according any one of claims 19 to 22, **characterized in that** the oligonucleotide consists of ribonucleotide or deoxyribonucleotide units, at least one of which may comprise a functional group introduced onto or generated on said unit, or the functional group introduced by using a spacer arm bonded to the terminal phosphate group in the 3' or 5' position.

**24.** The conjugate according to claim 23, **characterized in that** said unit is the 5' terminal unit or 3' terminal unit.

**25.** The conjugate according to any one of claims 19 to 24, **characterized in that** the oligonucleotide comprises a chain of 5 to 50 nucleotides or a chain of 5 to 50 nucleotides and nucleotide analogs or nucleoside analogs.

**26.** The conjugate according to any one of claims 19 to 25, **characterized in that** the oligonucleotide consists of a chain of ribonucleotide or deoxyribonucleotide units bonded to one another via phosphodiester-type bonds and of analogous units of nucleosides bonded to one another via amide bonds, said oligonucleotide comprising at least 5 phosphodiester-type internucleotide bonds at the end intended to be bonded to the cryptate.

**27.** The conjugate according to any one of claims 19 to 26, **characterized in that** the rare-earth metal cryptate is bonded covalently to the oligonucleotide either directly or via a spacer arm.

**28.** The conjugate according to any one of claims 19 to 27, **characterized in that** said rare-earth metal cryptate consists of at least one rare-earth metal salt complexed with a macropolycyclic compound of formula:

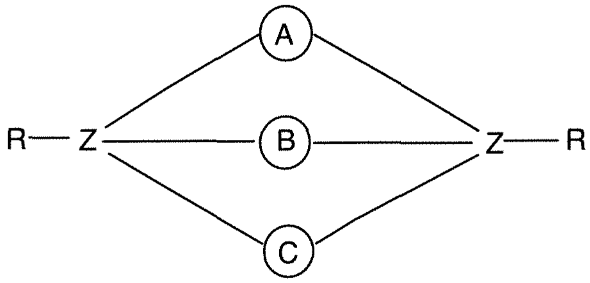

I

in which Z is an atom with 3 or 4 valencies, R is nothing or represents hydrogen, a hydroxy group, an amino group or a hydrocarbon-based radical, the divalent radicals Ⓐ, Ⓑ and Ⓒ, are, independently of each other, hydrocarbon-based chains which optionally contain one or more heteroatoms and are optionally interrupted with a heteromacrocycle, at least one of the radicals Ⓐ, Ⓑ and Ⓒ, also comprising at least one molecular unit or consisting essentially of a molecular unit, said molecular unit having a triplet energy which is greater than that of the emission level of the complexed rare-earth metal ion.

**29.** The conjugate according to any of claims 19 to 28, **characterized in that** the rare-earth metal cryptate consists of a rare-earth metal salt complexed with one of the macrocyclic or macropolycyclic compounds below:

(22)phenanthroline; (22)phenanthroline amide; (22)anthracene; (22)anthracene amide; (22)bi-isoquinoline; (22) biphenyl-bis-pyridine; (22)bipyridine; (22)bi-pyridine amide; the macropolycycles tris-bipyridine, tris-phenanthroline, phenanthroline-bis-bipyridine, bi-isoquinoline-bis-bipyridine, bis-bipyridine diphenylbipyridine; a macropolycyclic compound comprising a molecular unit chosen from bipyrazines, bipyrimidines and nitrogen-containing heterocycles comprising N-oxide groups.

**30.** The conjugate according to any one of claims 19 to 29, **characterized in that** the rare-earth metal cryptate consists of at least one rare-earth metal salt complexed with a macropolycyclic compound corresponding to one of the formulae II or III below:

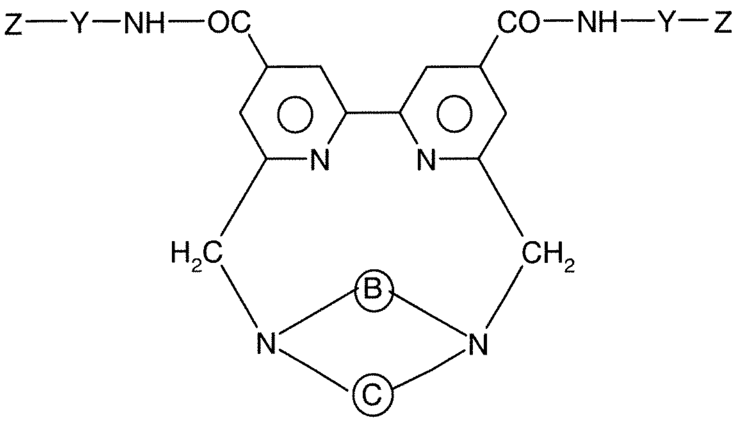

II

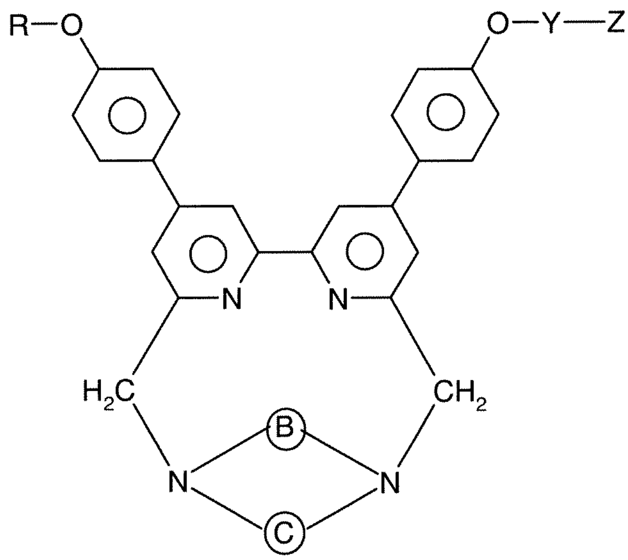

III

in which:

- the ring of formula

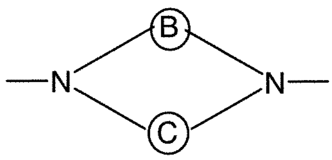

is one of the following rings:

1)

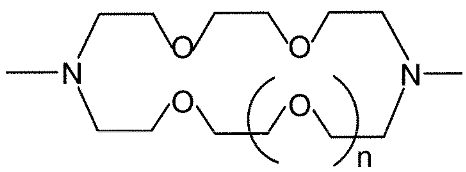

n = 0 or 1

$[N_2O_4]$ macrocycle or cycle (22)

$[N_2O_3]$ macrocycle or cycle (21)

2)

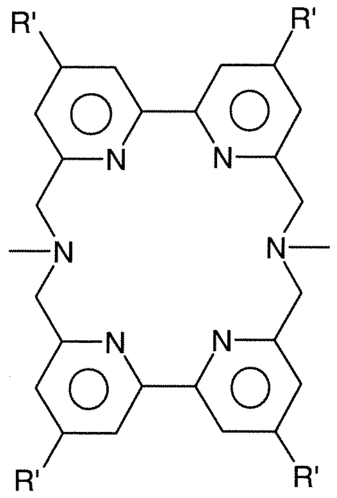

bis-bipyridine macrocylcle

- Y is a spacer group or spacer arm which consists of a divalent organic radical, chosen from linear or branched $C_1$ to $C_{20}$ alkylene groups optionally containing one or more double bonds and/or optionally containing one or more heteroatoms such as oxygen, nitrogen, sulfur or phosphorus or one or more carbamoyl or carboxamido group(s); chosen from $C_5$ to $C_8$ cycloalkylene groups or chosen from $C_6$ to $C_{14}$ arylene groups, said alkylene, cycloalkylene or arylene groups being optionally substituted with alkyl, aryl or sulfonate groups;
- Z is a functional group capable of bonding covalently to a biological substance;
- R is a methyl group or represents the group -Y-Z;
- R' is hydrogen or a group -COOR" in which R" is a $C_1$ to $C_{10}$ alkyl group and preferably represents a methyl, ethyl or tert-butyl group, or alternatively R' is a group -CO-NH-Y-Z.

**31.** The conjugate according to any one of claims 19 to 30, **characterized in that** the rare-earth metal cryptate is bonded to the oligonucleotide via a spacer arm consisting of a divalent organic radical chosen from $C_1$-$C_{20}$ linear or branched alkylene groups optionally containing one or more double bonds or triple bonds and/or optionally containing one or more hetero atoms, such as oxygen, nitrogen, sulfur, phosphorus or one or more carbamoyl or carboxamido group (s); $C_5$-$C_8$ cycloalkylene groups and $C_6$-$C_{14}$ arylene groups, said alkylene, cycloalkylene or arylene groups being optionally substituted with alkyl, aryl or sulfonate groups.

**32.** The conjugate according to any of claims 19 to 31, **characterized in that** the spacer arm is chosen from the groups:

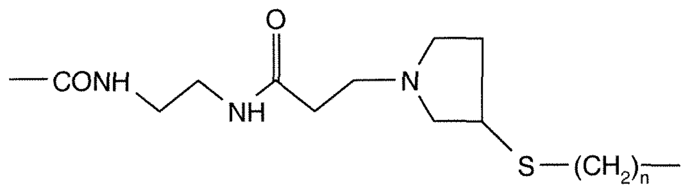

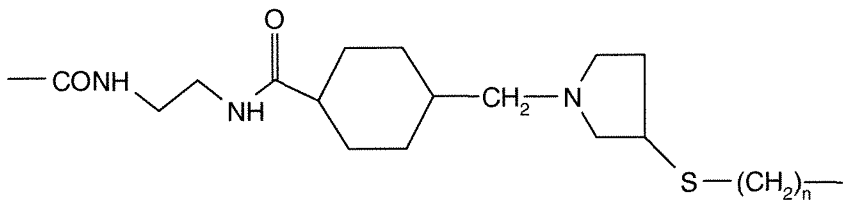

in which n = 2 to 6, and $-CONH-(CH_2)_6-$, the attachment via the group -CONH taking place on the cryptate.

**33.** The conjugate according to any of claims 19 to 32, **characterized in that** the rare-earth metal cryptate is a europium cryptate.

**34.** The conjugate according to any of claims 19 to 33, **characterized in that** the rare-earth metal cryptate is the europium cryptate Eu trisbipyridine or Eu [bis-diethoxybipyridine.bipyridine].

**Patentansprüche**

**1.** Verfahren zur Verminderung der Fluoreszenzlöschung bedingt durch ein Medium zur Messung in einer Dosierung durch Fluoreszenz von einem Analyten, das wenigstens einen Fluoreszenzmarker einsetzt, **dadurch gekennzeichnet, daß** man in das Meßmedium ein Fluoreszenzkonjugat einführt, umfassend ein Oligonukleotid, das gebunden ist an ein Seltenerdkryptat, und **dadurch**, daß das Konjugat kovalent gebunden ist an ein biologisches Molekül mit einer Erkennungsrolle und welches sich an einen Partner binden kann, wobei das biologische Molekül eines der Mitglieder eines Paares von Molekülen ist, die in der Lage sind, sich spezifisch untereinander zu binden, gewählt unter den folgenden Paaren: Paar Ligand/Rezeptor, Paar Antigen/Antikörper, Paar Biotin/Avidin, Paar Nukleinsäure/Nukleinsäure, mit der Bedingung, daß, wenn das biologische Molekül eine Nukleinsäure ist, das Oligonukleotid gewählt ist unter den folgenden Oligonukleotiden: -$^{(AH)}$C ACG CCA CTA GCT CC-, -$^{(AH)}$C GGG GGT TTT TTT TTT-, -$^{(AH)}$GGG GGT TTT TTT TT$^{(MCC-AH)}$ CT-, -TTT TTT TTT GGG GG$^{(AH)}$CG-.

**2.** Verfahren nach Anspruch 1, **dadurch gekennzeichnet, daß** das Oligonukleotid besteht aus einer Aneinanderkettung von Ribonukleotid- oder Desoxyribonukleotideinheiten, die untereinander durch Bindungen vom Phosphodiestertyp verbunden sind.

**3.** Verfahren nach einem der Ansprüche 1 oder 2, **dadurch gekennzeichnet, daß** das Oligonukleotid besteht aus einer Aneinanderkettung von Ribonukleotid- oder Desoxyribonukleotideinheiten oder von Nukleotidanalogaeinheiten, die auf dem Zucker oder auf der Base modifiziert sind und untereinander verbunden sind durch natürliche Internukleotidbindungen vom Phosphodiestertyp, wobei ein Teil der Internukleotidbindungen gegebenenfalls ersetzt ist durch Phosphat-, Phosphoamid- oder Phosphothioatbindungen.

**4.** Verfahren nach einem der Ansprüche 1 oder 2, **dadurch gekennzeichnet, daß** das Oligonukleotid besteht aus einer Aneinanderkettung, umfassend gleichzeitig Ribonukleotid- oder Desoxyribonukleotideinheiten, die untereinander verbunden sind durch Bindungen vom Phosphodiestertyp, und Nukleosidanalogaeinheiten, die untereinander verbunden sind durch Amidbindungen.

**5.** Verfahren nach einem der Ansprüche 1 bis 4, **dadurch gekennzeichnet, daß** das Oligonukleotid besteht aus Ribonukleotid- oder Desoxyribonukleotideinheiten, von denen wenigstens eine eine funktionelle Gruppe umfassen kann, die eingeführt oder erzeugt ist auf der Einheit, oder eine funktionelle Gruppe, die eingeführt ist mit Hilfe eines Beabstandungsarms, der gebunden ist an die terminale Phosphatgruppe in 3'-oder 5'-Position.

**6.** Verfahren nach Anspruch 5, **dadurch gekennzeichnet, daß** die Einheit die 5'-terminale oder 3'-terminale Einheit ist.

**7.** Verfahren nach einem der Ansprüche 1 bis 6, **dadurch gekennzeichnet, daß** das Oligonukleotid eine Aneinanderkettung von 5 bis 50 Nukleotiden oder eine Aneinanderkettung von 5 bis 50 Nukleotiden und Nukleotidanaloga oder Nukleosidanaloga umfaßt.

**8.** Verfahren nach einem der Ansprüche 1 bis 6, **dadurch gekennzeichnet, daß** das Oligonukleotid besteht aus einer Aneinanderkettung von Ribonukleotid- oder Desoxyribonukleotideinheiten, die untereinander verbunden sind durch Bindungen vom Phosphodiestertyp, und Nukleosidanalogaeinheiten, die untereinander verbunden sind durch Amidbindungen, wobei das Oligonukleotid wenigstens 5 Internukleotidbindungen vom Phosphodiestertyp an dem Ende hat, das vorgesehen ist, an das Kryptat gebunden zu werden.

**9.** Verfahren nach einem der Ansprüche 1 bis 8, **dadurch gekennzeichnet, daß** das Seltenerdkryptat kovalent gebunden ist an das Oligonukleotid, entweder direkt oder mit einem Beabstandungsarm.

**10.** Verfahren nach einem der Ansprüche 1 bis 9, **dadurch gekennzeichnet, daß** das Seltenerdkryptat besteht aus wenigstens einem Seltenerdsalz, das komplexiert ist durch eine makropolyzyklische Verbindung mit einer Formel

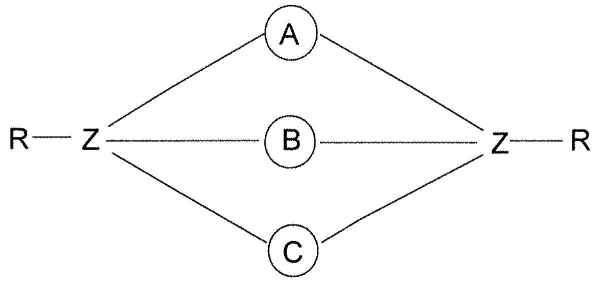

I

in welcher Z ein Atom mit 3 oder 4 Valenzen ist, R nichts ist oder ein Wasserstoff, die Hydroxygruppe, eine Aminogruppe oder einen Kohlenwasserstoffrest darstellt, die bivalenten Radikale Ⓐ, Ⓑ und Ⓒ unabhängig voneinander Kohlenwasserstoffketten sind, die gegebenenfalls eines oder mehrere Heteroatome enthalten und gegebenenfalls unterbrochen sind durch einen Heteromakrozyklus, wobei wenigstens eines der Radikale Ⓐ, Ⓑ und Ⓒ weiterhin wenigstens ein Molekülmotiv umfaßt oder im wesentlichen besteht aus einem Molekülmotiv, wobei das Molekülmotiv eine Triplettenergie höher als jene des Emissionsniveaus des komplexierten Seltenerdions aufweist.

**11.** Verfahren nach Anspruch 10, **dadurch gekennzeichnet, daß** das Seltenerdkryptat besteht aus einem Seltenerdsalz, das komplexiert ist durch eine der nachfolgenden makrozyklischen oder makropolyzyklischen Verbindungen: (22)Phenanthrolin; (22)Phenanthrolinamid; (22)Anthracen; (22)Anthracenamid; (22)Biisochinolin; (22)Biphenyl-bis-Pyridin; (22)Bipyridin; (22)Bipyridinamid; die Makropolyzyklen Tris-Bipyridin, Tris-Phenanthrolin, Phenanthrolin-bis-Bipyridin, BiIsochinolin-bis-Bipyridin, Bis-Bipyridin-Diphenylbipyridin; eine makropolyzyklische Verbindung, die ein Molekülmotiv umfaßt, das gewählt ist unter den Bipyrazinen, den Bipyrimidinen und den stickstoffhaltigen Heterozyklen, welche N-Oxidgruppen umfassen.

**12.** Verfahren nach einem der Ansprüche 1 bis 9, **dadurch gekennzeichnet, daß** das Seltenerdkryptat besteht aus wenigstens einem Seltenerdsalz, das komplexiert ist durch eine makropolyzyklische Verbindung, die einer der nachfolgenden Formeln II oder III entspricht:

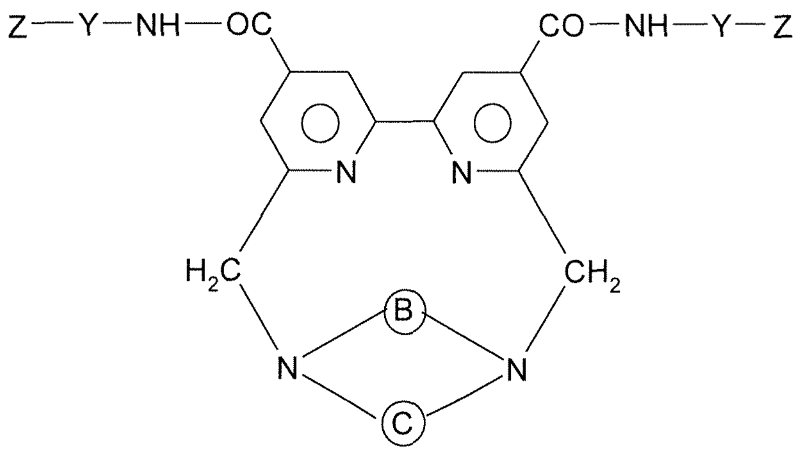

II

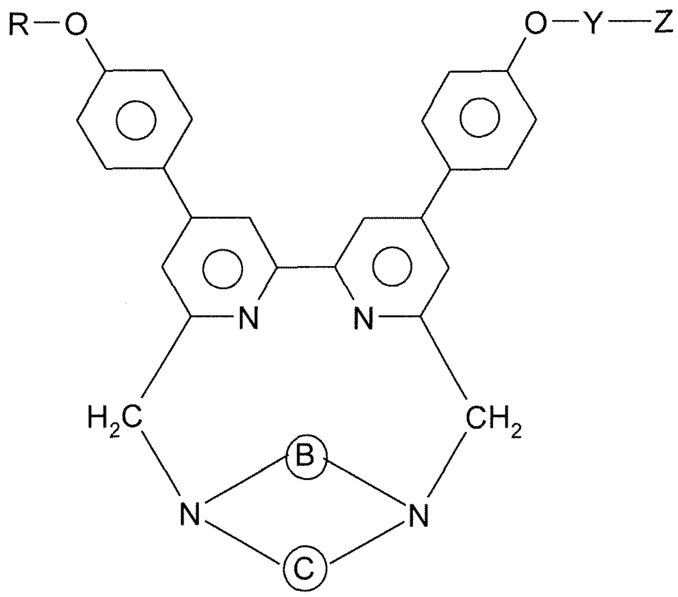

III

in denen:

- der Zyklus mit einer Formel

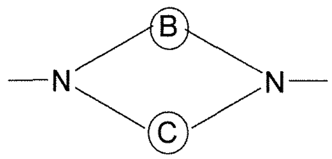

einer der folgenden Zyklen ist:

1)

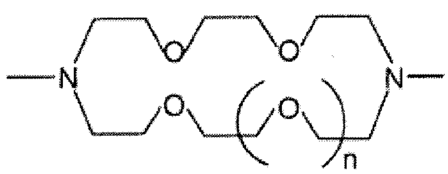

n = 0 oder 1
Makrozyklus [$N_2O_4$] oder Zyklus (22)
Makrozyklus [$N_2O_3$] oder Zyklus (21)

2)

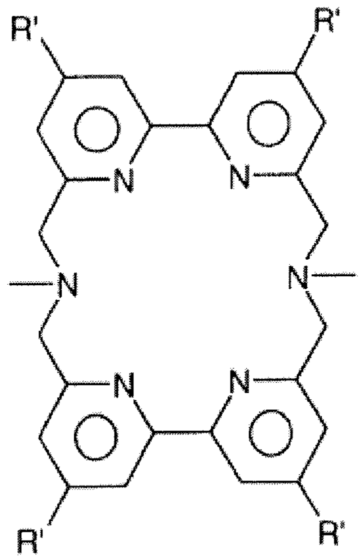

Bis-Bipyridin-Makrozyklus

- Y eine Gruppe oder ein Beabstandungsarm ist, die/der besteht aus einem bivalenten organischen Rest, gewählt unter den linearen oder verzweigten $C_1$- bis $C_{20}$-Alkylengruppen, die gegebenenfalls eine oder mehrere Doppelbindungen enthalten und/oder gegebenenfalls eines oder mehrere Heteroatome enthalten, wie Sauerstoff, Stickstoff, Schwefel, Phosphor oder eine oder mehrere Carbamoyl- oder Carboxamidgruppe(n); unter den $C_5$- bis $C_8$-Zykloalkylengruppen oder unter den $C_6$- bis $C_{14}$-Arylengruppen, wobei die Alkylen-, Zykloalkylen- oder Arylengruppen gegebenenfalls substituiert sind durch Alkyl-, Aryl- oder Sulfonatgruppen;
- Z eine funktionelle Gruppe ist, die geeignet ist, sich in kovalenter Weise mit einer biologischen Substanz zu verbinden;
- R eine Methylgruppe ist oder die Gruppe -Y-Z darstellt; R' Wasserstoff oder eine -COOR"-Gruppe ist, in welcher R" eine $C_1$- bis $C_{10}$-Alkylgruppe ist und vorzugsweise eine Methyl-, Ethyl- oder tert.-Butylgruppe darstellt oder auch R' eine Gruppe -CO-NH-Y-Z ist.

13. Verfahren nach einem der Ansprüche 1 bis 12, **dadurch gekennzeichnet, daß** das Seltenerdkryptat an das Oligonukleotid mit einem Beabstandungsarm gebunden ist, der besteht aus einem bivalenten organischen Rest, gewählt unter den linearen oder verzweigten $C_1$- bis $C_{20}$-Alkylengruppen, die gegebenenfalls eine oder mehrere Doppel- oder Dreifachbindungen enthalten und/oder gegebenenfalls eines oder mehrere Heteroatome enthalten wie Sauerstoff, Stickstoff, Schwefel, Phosphor oder eine oder mehrere Carbamoyl- oder Carboxamidgruppe(n), den $C_5$-$C_8$-Zykloalkylengruppen und die $C_6$-$C_{14}$-Arylengruppen, wobei die Alkylen-, Zykloalkylen- oder Arylengruppen gegebenenfalls substituiert sind durch Alkyl-, Aryl- oder Sulfonatgruppen.

14. Verfahren nach Anspruch 13, **dadurch gekennzeichnet, daß** der Beabstandungsarm gewählt ist unter den Gruppen:

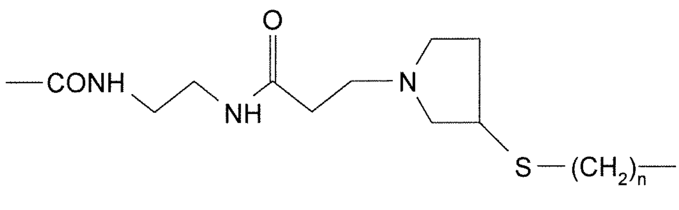

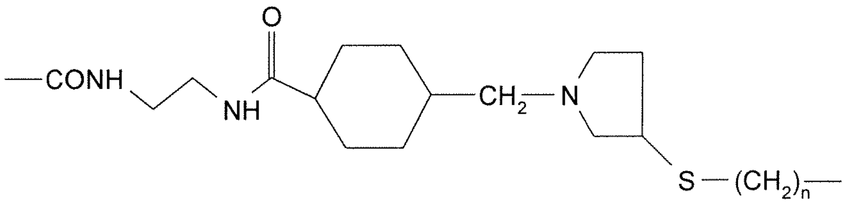

in welchen n = 2 bis 6, und -CONH-$(CH_2)_6$-, wobei die Bindung über die Gruppe -CONH auf der Ebene des Kryptats stattfindet.

**15.** Verfahren nach einem der Ansprüche 1 bis 14, **dadurch gekennzeichnet, daß** das Seltenerdkryptat ein Europiumkryptat ist.

**16.** Verfahren nach Anspruch 15, **dadurch gekennzeichnet, daß** das Seltenerdkryptat das Europiumkryptat Eu-Trisbipyridin oder Eu[bis-Diethoxybipyridin.Bipyridin] ist.

**17.** Verfahren nach einem der Ansprüche 1 bis 16, **dadurch gekennzeichnet, daß** das Fluoreszenzkonjugat verwendet wird als alleiniger Marker oder als einer der Fluoreszenzmarker in der Dosierung.

**18.** Verfahren nach einem der Ansprüche 1 bis 17, **dadurch gekennzeichnet, daß** man zusätzlich zu dem Fluoreszenzkonjugat in der Dosierung einen Fluoreszenzmarker einsetzt, der eine Fluoreszenzakzeptorverbindung umfaßt.

**19.** Fluoreszenzkonjugat, **dadurch gekennzeichnet, daß** es umfaßt:

(i) ein Seltenerdkryptat,
(ii) ein Oligonukleotid,
(iii) ein biologisches Molekül mit einer Erkennungsrolle und welches sich an einen Partner binden kann, wobei das biologische Molekül eines der Mitglieder eines Paares von Molekülen ist, die sich spezifisch untereinander verbinden können, gewählt unter den folgenden Paaren: Paar Ligand/Rezeptor, Paar Antigen/Antikörper, Paar Biotin/Avidin, Paar Nukleinsäure/Nukleinsäure, mit der Bedingung, daß, wenn das biologische Molekül eine Nukleinsäure ist, das Oligonukleotid gewählt ist unter den folgenden Oligonukleotiden: -$^{(AH)}$C ACG CCA CTA GCT CC-, -$^{(AH)}$C GGG GGT TTT TTT TTT-, -$^{(AH)}$GGG GGT TTT TTT TT$^{(MCC\text{-}AH)}$CT-, -TTT TTT TTT GGG GG$^{(AH)}$CG-, wobei (i), (ii) und (iii) durch kovalente Bindungen verbunden sind.

**20.** Konjugat nach Anspruch 19, **dadurch gekennzeichnet, daß** das Oligonukleotid besteht aus einer Aneinanderkettung von Ribonukleotid- oder Desoxyribonukleotideinheiten, die untereinander durch Bindungen vom Phosphodiestertyp verbunden sind.

**21.** Konjugat nach einem der Ansprüche 19 oder 20, **dadurch gekennzeichnet, daß** das Oligonukleotid besteht aus einer Aneinanderkettung von Ribonukleotid- oder Desoxyribonukleotideinheiten oder von Nukleotidanalogaeinheiten, die auf dem Zucker oder auf der Base modifiziert sind und untereinander verbunden sind durch natürliche Internukleotidbindungen vom Phosphodiestertyp, wobei ein Teil der Internukleotidbindungen gegebenenfalls ersetzt ist durch Phosphonat-, Phosphoamid- oder Phosphothioatbindungen.

**22.** Konjugat nach einem der Ansprüche 19 oder 20, **dadurch gekennzeichnet, daß** das Oligonukleotid besteht aus einer Aneinanderkettung, umfassend gleichzeitig Ribonukleotid- oder Desoxyribonukleotideinheiten, die untereinander verbunden sind durch Bindungen vom Phosphodiestertyp, und Nukleosidanalogaeinheiten, die untereinander verbunden sind durch Amidbindungen.

**23.** Konjugat nach einem der Ansprüche 19 bis 22, **dadurch gekennzeichnet, daß** das Oligonukleotid besteht aus Ribonukleotid- oder Desoxyribonukleotideinheiten, von denen wenigstens eine eine funktionelle Gruppe umfassen kann, die eingeführt oder erzeugt ist auf der Einheit, oder eine funktionelle Gruppe, die eingeführt ist mit Hilfe eines Beabstandungsarms, der gebunden ist an die terminale Phosphatgruppe in 3'-oder 5'-Position.

**24.** Konjugat nach Anspruch 23, **dadurch gekennzeichnet, daß** die Einheit die 5'-terminale oder 3'-terminale Einheit ist.

**25.** Konjugat nach einem der Ansprüche 19 bis 24, **dadurch gekennzeichnet, daß** das Oligonukleotid eine Aneinanderkettung von 5 bis 50 Nukleotiden oder eine Aneinanderkettung von 5 bis 50 Nukleotiden und Nukleotidanaloga oder Nukleosidanaloga umfaßt.

**26.** Konjugat nach einem der Ansprüche 19 bis 25, **dadurch gekennzeichnet, daß** das Oligonukleotid besteht aus einer Aneinanderkettung von Ribonukleotid- oder Desoxyribonukleotideinheiten, die untereinander verbunden sind durch Bindungen vom Phosphodiestertyp, und Nukleosidanalogaeinheiten, die untereinander verbunden sind durch Amidbindungen, wobei das Oligonukleotid wenigstens 5 Internukleotidbindungen vom Phosphodiestertyp an dem Ende hat, das vorgesehen ist, an das Kryptat gebunden zu werden.

**27.** Konjugat nach einem der Ansprüche 19 bis 26, **dadurch gekennzeichnet, daß** das Seltenerdkryptat kovalent gebunden ist an das Oligonukleotid, entweder direkt oder mit einem Beabstandungsarm.

**28.** Konjugat nach einem der Ansprüche 19 bis 27, **dadurch gekennzeichnet, daß** das Seltenerdkryptat besteht aus wenigstens einem Seltenerdsalz, das komplexiert ist durch eine makropolyzyklische Verbindung mit einer Formel

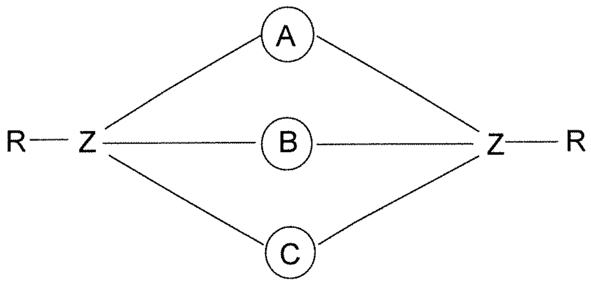

I

in welcher Z ein Atom mit 3 oder 4 Valenzen ist, R nichts ist oder ein Wasserstoff, die Hydroxygruppe, eine Aminogruppe oder einen Kohlenwasserstoffrest darstellt, die bivalenten Radikale Ⓐ, Ⓑ und Ⓒ unabhängig voneinander Kohlenwasserstoffketten sind, die gegebenenfalls eines oder mehrere Heteroatome enthalten und gegebenenfalls unterbrochen sind durch einen Heteromakrozyklus, wobei wenigstens eines der Radikale Ⓐ, Ⓑ und Ⓒ weiterhin wenigstens ein Molekülmotiv umfaßt oder im wesentlichen besteht aus einem Molekülmotiv, wobei das Molekülmotiv eine Triplettenergie höher als jene des Emissionsniveaus des komplexierten Seltenerdions aufweist.

**29.** Konjugat nach einem der Ansprüche 19 bis 28, **dadurch gekennzeichnet, daß** das Seltenerdkryptat besteht aus einem Seltenerdsalz, das komplexiert ist durch eine der nachfolgenden makrozyklischen oder makropolyzyklischen Verbindungen: (22)Phenanthrolin; (22)Phenanthrolinamid; (22)Anthracen; (22)Anthracenamid; (22)Biisochinolin; (22)Biphenyl-bis-Pyridin; (22)Bipyridin; (22)Bipyridinamid; die Makropolyzyklen Tris-Bipyridin, Tris-Phenanthrolin, Phenanthrolin-bis-Bipyridin, BiIsochinolin-bis-Bipyridin, Bis-Bipyridin-Diphenylbipyridin; eine makropolyzyklische Verbindung, die ein Molekülmotiv umfaßt, das gewählt ist unter den Bipyrazinen, den Bipyrimidinen und den stickstoffhaltigen Heterozyklen, welche N-Oxidgruppen umfassen.

**30.** Konjugat nach einem der Ansprüche 19 bis 29, **dadurch gekennzeichnet, daß** das Seltenerdkryptat besteht aus wenigstens einem Seltenerdsalz, das komplexiert ist mit einer makropolyzyklischen Verbindung, die einer der Formeln II oder III hiernach entspricht:

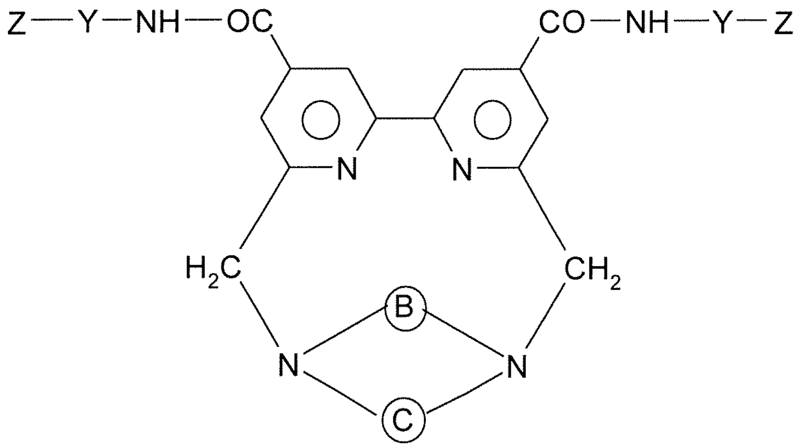

II

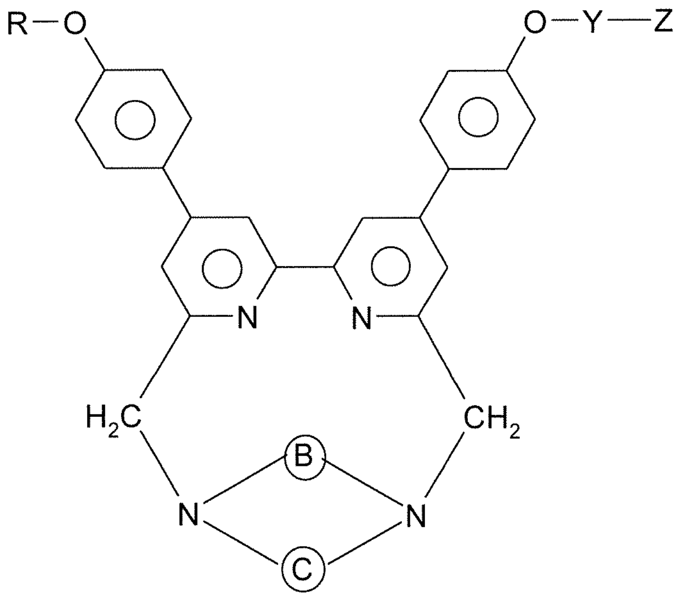

III

in denen:

der Zyklus mit einer Formel

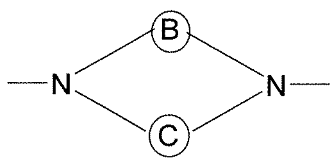

einer der folgenden Zyklen ist:

1)

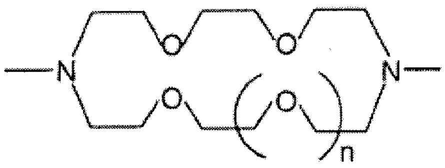

n = 0 oder 1
Makrozyklus [$N_2O_4$] oder Zyklus (22)
Makrozyklus [$N_2O_3$] oder Zyklus (21)

2)

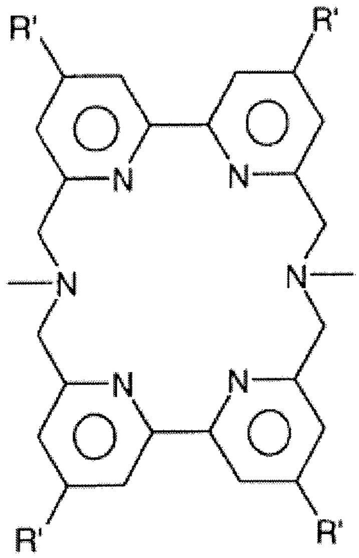

Bis-Bipyridin-Makrozyklus

Y eine Gruppe oder ein Beabstandungsarm ist, die/der besteht aus einem bivalenten organischen Rest, der gewählt ist unter den linearen oder verzweigten $C_1$- bis $C_{20}$-Alkylengruppen, die gegebenenfalls eine oder mehrere Doppelbindungen enthalten und/oder gegebenenfalls eines oder mehrere Heteroatome enthalten, wie Sauerstoff, Stickstoff, Schwefel, Phosphor oder eine oder mehrere Carbamoyl- oder Carboxamidgruppe(n); unter den $C_5$- bis $C_8$-Zykloalkylengruppen oder unter den $C_6$- bis $C_{14}$-Arylengruppen, wobei die Alkylen-, Zykloalkylen- oder Arylengruppen gegebenenfalls substituiert sind durch Alkyl-, Aryl- oder Sulfonatgruppen;
Z eine funktionelle Gruppe ist, die geeignet ist, sich in kovalenter Weise mit einer biologischen Substanz zu verbinden;
R eine Methylgruppe ist oder die Gruppe -Y-Z darstellt;
R' Wasserstoff oder eine -COOR"-Gruppe ist, in welcher R" eine $C_1$- bis $C_{10}$-Alkylengruppe ist und vorzugsweise eine Methyl-, Ethyl- oder tert.-Butylgruppe darstellt oder auch R' eine -CO-NH-Y-Z-Gruppe ist.

**31.** Konjugat nach einem der Ansprüche 19 bis 30, **dadurch gekennzeichnet, daß** das Seltenerdkryptat an das Oligonukleotid mit einem Beabstandungsarm gebunden wird, der besteht aus einem bivalenten organischen Rest, der gewählt ist unter den linearen oder verzweigten $C_1$-$C_{20}$-Alkylengruppen, die gegebenenfalls eine oder mehrere Doppel- oder Dreifachbindungen enthalten und/oder gegebenenfalls eines oder mehrere Heteroatome enthalten wie Sauerstoff, Stickstoff, Schwefel, Phosphor oder eine oder mehrere Carbamoyl- oder Carboxamidgruppe(n), den $C_5$- bis $C_8$-Zykloalkylengruppen und die $C_6$- bis $C_{14}$-Arylengruppen, wobei die Alkylen-, Zykloalkylen- oder Arylengruppen gegebenenfalls substituiert sind durch Alkyl-, Aryl- oder Sulfonatgruppen.

**32.** Konjugat nach einem der Ansprüche 19 bis 31, **dadurch gekennzeichnet, daß** der Beabstandungsarm gewählt ist unter den Gruppen:

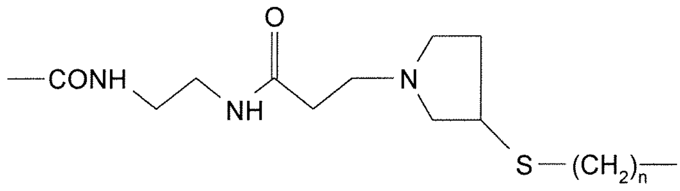

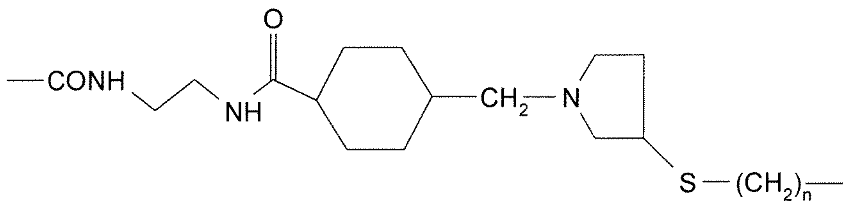

in welchen n = 2 bis 6, und $-CONH-(CH_2)_6-$, wobei die Bindung über die Gruppe -CONH auf der Ebene des Kryptats stattfindet.

33. Konjugat nach einem der Ansprüche 19 bis 32, **dadurch gekennzeichnet, daß** das Seltenerdkryptat ein Europiumkryptat ist.

34. Konjugat nach einem der Ansprüche 19 bis 33, **dadurch gekennzeichnet, daß** das Seltenerdkryptat das Europiumkryptat Eu-Trisbipyridin oder Eu[bis-Diethoxybipyridin.Bipyridin] ist.

# Figure 1

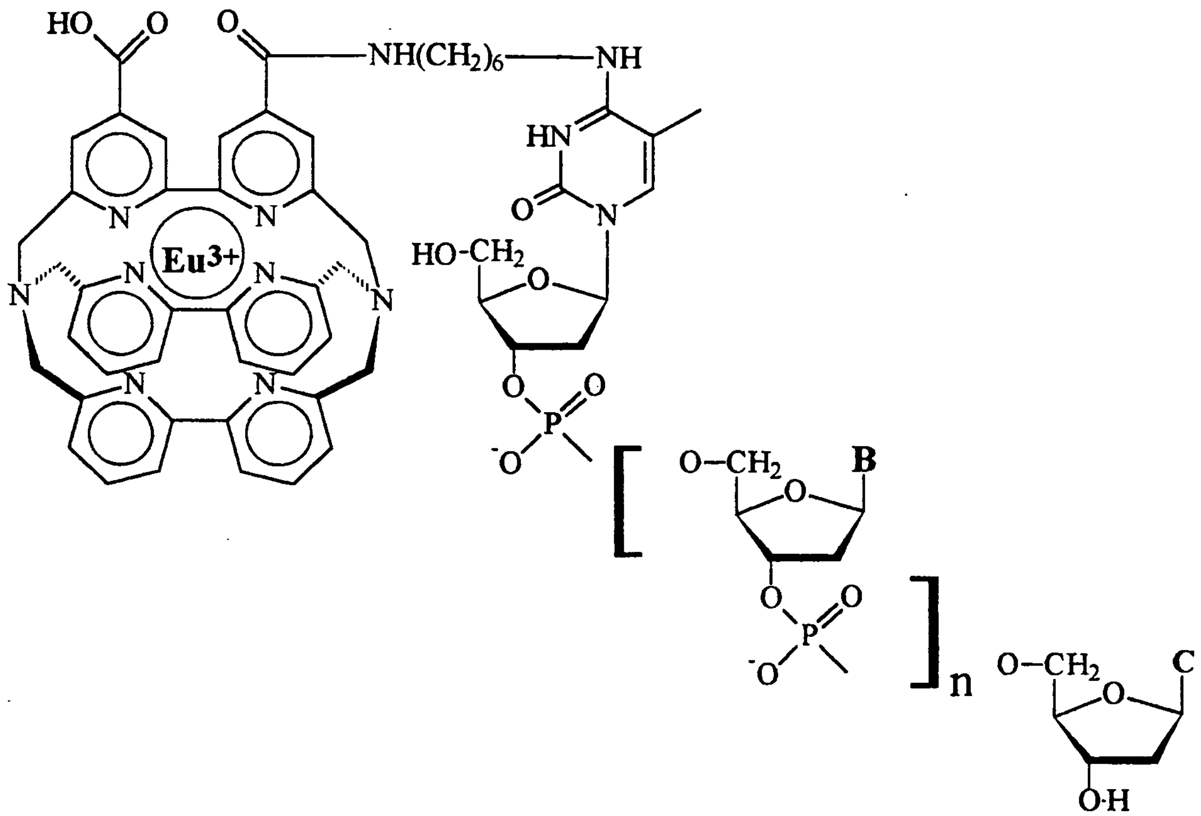

Conjugué KH-ODN1

B = Adénine (A), Guanine (G), Cytosine (C), Thymine (T)

C = Cytosine

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0321353 A
- WO 9201124 A
- EP 180492 A
- EP 00910933 A
- FR 9903150


### Littérature non-brevet citée dans la description


- **1. HEMMILÄ.** *Clin. Chem.,* 1985, vol. 31/3, 359-370
- **E. LOPEZ et al.** *Clin. Chem.,* 1993, vol. 39/2, 196-201
- **G.MATHIS et al.** *Clin. Chem,* 1993, vol. 39, 1251
- **J.M. LEHN.** *Struct. Bonding,* 1973, vol. 16, 1
- *Acc. Chem. Res.,* 1978, vol. 11, 49
- **GOODCHILD.** *Bioconjugate Chemistry,* Mai 1990, vol. 1 (3), 77-99
- **M. EGHOLM et al.** *J. Am. Chem. Soc.,* 1992, vol. 114, 1895-1897
- **R. VINAYAK et al.** *Nucleoside & Nucleotide,* 1997, vol. 16 (7-9), 1653-1656
- **F. BODAR-HOUILLON et al.** *New J. Chem.,* 1996, vol. 20, 1041-1045
- **J. M. LEHN et al.** *Helv. Chim. Acta,* 1992, vol. 75, 1221
- **J.M. LEHN et al.** *Helv. Chim. Acta,* 1991, vol. 74, 572
- **C. KESSLER.** Nonisotopic probing, Blotting and Sequencing. Academic press Ltd, 1995, 66-72
- **G. MATHIS et al.** *Clin. Chem.,* 1993, vol. 39, 1953-1959
- **ROGET et al.** *Nucleic Acids Res.,* 1989, vol. 17, 7643-7650

LISTAGE DES SEQUENCES.txt

<110> CIS BIO INTERNATIONAL
MATHIS, Gérard
BAZIN, Hervé
TRINQUET, Eric

<120> Réduction de l'extinction de fluorescence lors d'un dosage

<130> 1H189130 0016 EP ECT

<140> EP 00 910 933.1
<141> 2000-03-14

<150> FR 99 03150
<151> 1999-03-15

<160> 9

<170> PatentIn version 3.3

<210> 1
<211> 15
<212> DNA
<213> Artificial

<220>
<223> oligonucléotide

<220>
<221> misc_difference
<222> (1)..(1)
<223> cytosine modifiée par un bras aminohexyl (AH)

<400> 1
cacgccacta gctcc 15

<210> 2
<211> 15
<212> DNA
<213> Artificial

<220>
<223> oligonucléotide

<220>
<221> misc_difference
<222> (1)..(1)
<223> cytosine modifiée par un bras aminohexyl (AH) puis couplée à un cryptate (K)

<400> 2
cacgccacta gctcc 15

<210> 3
<211> 16
<212> DNA
<213> Artificial

<220>
<223> oligonucléotide

<220>
<221> misc_difference
<222> (1)..(1)
<223> cytosine modifiée par un bras aminohexyl (AH)

<400> 3
cgggggtttt tttttt 16

<210> 4
<211> 16
<212> DNA
<213> Artificial

<220>
<223> oligonucléotide

<220>
<221> misc_difference
<222> (1)..(1)
<223> cytosine modifiée par un bras aminohexyl (AH) puis couplée à un cryptate (K)

<400> 4
cgggggtttt tttttt 16

<210> 5
<211> 16

<212> DNA
<213> Artificial

<220>
<223> oligonucléotide

<220>
<221> misc_difference
<222> (1)..(1)
<223> guanine modifiée par un bras aminohexyl (AH) puis couplée à un cryptate (K)

<220>
<221> misc_difference
<222> (15)..(15)
<223> cytosine modifiée par un bras aminohexyl (AH) portant un groupe réactif maléimide (MCC)

<400> 5
gggggttttt ttttct 16

<210> 6
<211> 16
<212> DNA
<213> Artificial

<220>
<223> oligonucléotide

<220>
<221> misc_difference
<222> (1)..(1)
<223> guanine modifiée par un bras aminohexyl (AH) sous sa forme protégée par un groupe monométhoxytrityl (MMT)

<220>
<221> misc_difference
<222> (15)..(15)
<223> cytosine modifiée par un bras aminohexyl (AH)

<400> 6
gggggttttt ttttct 16

<210> 7
<211> 16
<212> DNA
<213> Artificial

<220>
<223> oligonucléotide

<220>
<221> misc_difference
<222> (1)..(1)
<223> guanine modifiée par un bras aminohexyl (AH) sous sa forme protégée par un groupe monométhoxytrityl (MMT)

<220>
<221> misc_difference
<222> (15)..(15)

<223> cytosine modifiée par un bras aminohexyl (AH) portant un groupe réactif maléimide (MCC)

<400> 7
gggggttttt ttttct 16

<210> 8
<211> 16
<212> DNA
<213> Artificial

<220>
<223> oligonucléotide

<220>
<221> misc_difference
<222> (1)..(1)
<223> guanine modifiée par un bras aminohexyl (AH)

<220>
<221> misc_difference
<222> (15)..(15)
<223> cytosine modifiée par un bras aminohexyl (AH) portant un groupe réactif maléimide (MCC)

<400> 8
gggggttttt ttttct 16

<210> 9
<211> 16
<212> DNA
<213> Artificial

<220>
<223> oligonucléotide

<220>
<221> misc_difference
<222> (15)..(15)
<223> cytosine modifiée par un bras aminohexyl (AH)

<400> 9
tttttttttg ggggcg 16